(19) Europäisches Patentamt

European Patent Office

Office européen des brevets

(11) **EP 4 413 922 A1**

(12) **EUROPEAN PATENT APPLICATION**
published in accordance with Art. 153(4) EPC

(43) Date of publication:
**14.08.2024  Bulletin 2024/33**

(21) Application number: **22878449.2**

(22) Date of filing: **30.09.2022**

(51) International Patent Classification (IPC):
**A61B 5/11** (2006.01)     **A61B 5/0245** (2006.01)
**A61B 5/08** (2006.01)     **A61B 5/113** (2006.01)

(52) Cooperative Patent Classification (CPC):
**A61B 5/0245; A61B 5/0507; A61B 5/08;
A61B 5/11; A61B 5/113; A61B 8/02**

(86) International application number:
**PCT/JP2022/036795**

(87) International publication number:
**WO 2023/058580 (13.04.2023 Gazette 2023/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **04.10.2021   JP 2021163311**

(71) Applicant: **Nisshinbo Singapore Pte. Ltd.
Singapore Land Tower, 048623 (SG)**

(72) Inventors:
• **SAITO, Kazuo
  Singapore Pte. Ltd., 50 Raffles Place,  08-06
  Singapore Land Tower, 048623 (SG)**
• **TSUSHIMA, Kengo
  Mitaka-shi, Tokyo 181-0002 (JP)**
• **FUJII, Minori
  Singapore Pte. Ltd., 50 Raffles Place,  08-06
  Singapore Land Tower, 048623 (SG)**

(74) Representative: **v. Bezold & Partner Patentanwälte
- PartG mbB
Ridlerstraße 57
80339 München (DE)**

(54) **HEARTBEAT AND RESPIRATION DETECTION DEVICE AND HEARTBEAT AND RESPIRATION DETECTION PROGRAM**

(57)     In this disclosure, at heartbeat detection, a frequency component caused by a micro vibration of heartbeat is extracted over a predefined heartbeat observation time window, an amplitude peak is extracted from the frequency component caused by the micro vibration of the heartbeat, and one characteristic micro vibration out of a plurality of characteristic micro vibrations in one heartbeat is extracted. On the other hand, at respiration detection, positive and negative frequency components caused by the micro vibration of the heartbeat are extracted, complex multiplication of the positive and negative frequency components is performed, and from complex multiplication of the positive and negative frequency components, a respiration phase change caused by a micro vibration of respiration is extracted, with a heartbeat phase change caused by the micro vibration of the heartbeat removed.

FIG. 4

**Description**

TECHNICAL FIELD

This disclosure relates to a technique of calculating a heart rate and a respiration rate while enabling the alleviation of burdens on nurses and infection risk reduction based on radar signals or ultrasound signals reflecting off a body surface.

BACKGROUND ART

Patent Literature 1 and the like disclose a technique of calculating a heart rate and a respiration rate while enabling the alleviation of burdens on nurses and infection risk reduction based on radar signals reflecting off a body surface.

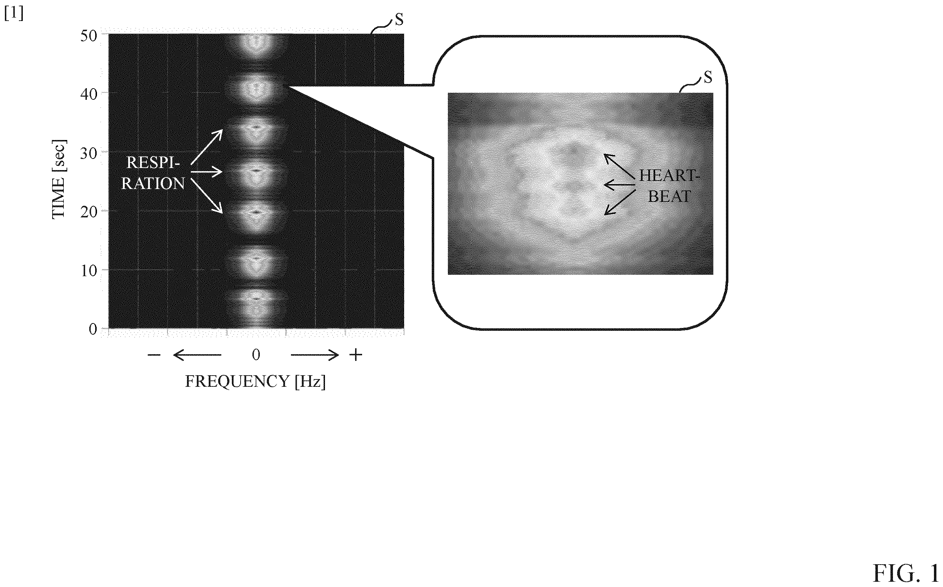

Fig. 1 illustrates an outline of heartbeat and respiration detection processing of the prior art. From radar signals reflecting off a body surface, a spectrogram S that shows a temporal change of each frequency component is calculated. From the spectrogram S, a direct current (DC) component including an extremely low frequency component is extracted. About once or twice in 10 seconds, amplitude peaks caused by a micro vibration of respiration are extracted. Based on a time interval of the respiration amplitude peaks, a respiration rate is calculated. About three times between the respiration amplitude peaks, amplitude peaks caused by a micro vibration of heartbeat are extracted. Based on a time interval of the heartbeat amplitude peaks, a heart rate is calculated.

CITATION LIST

Patent Literature

Patent Literature 1: JP-A-2019-129996

SUMMARY OF INVENTION

Technical Problem

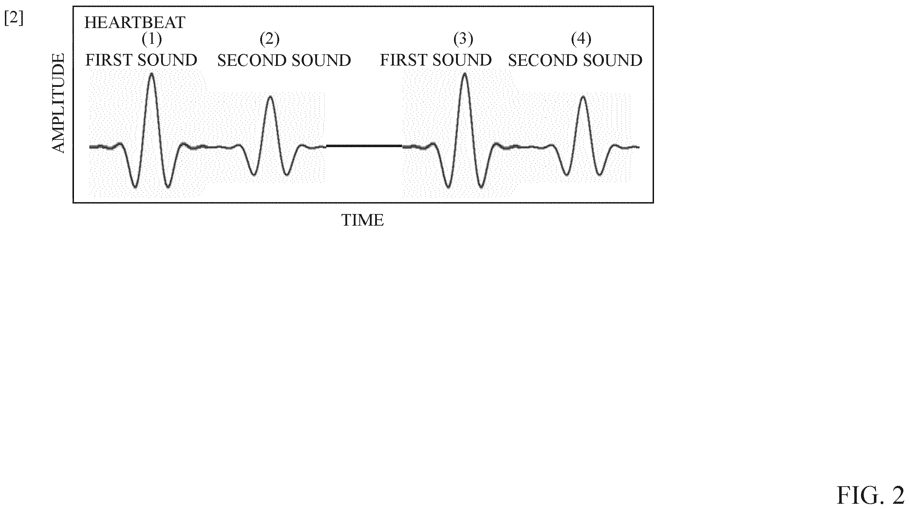

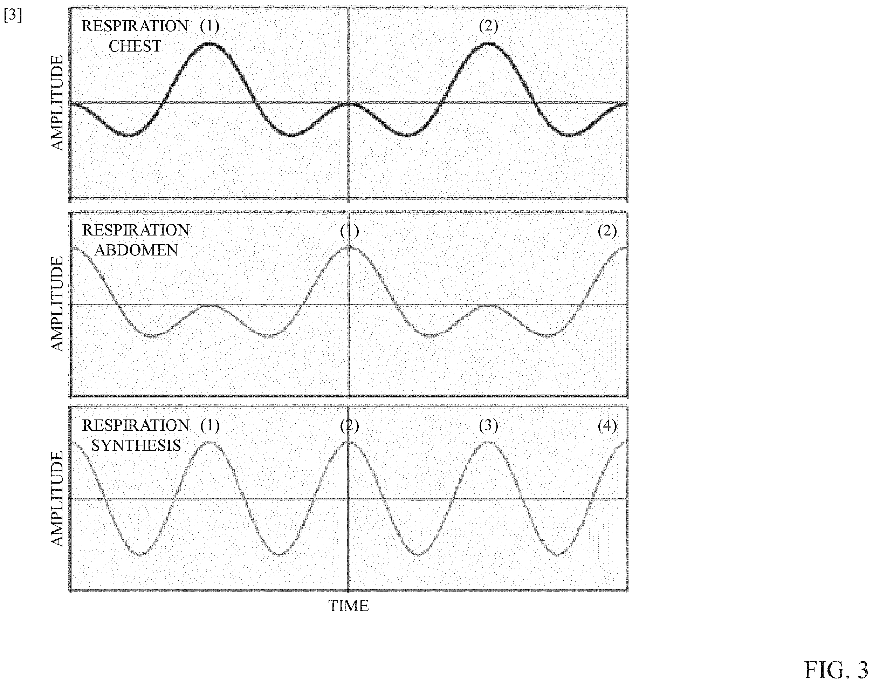

Fig. 1 illustrates the DC component including an extremely low frequency component being extracted from the spectrogram S. Since disturbances, such as louvers of air conditioners, curtains swayed by the wind and the movements of nurses, are mainly included in this extremely low frequency component and are close to a frequency component caused by the micro vibration of the respiration, it is difficult to ensure the improvement of robustness and the reduction of the effects of disturbances. Then, since the heartbeat amplitude peaks are small compared with the respiration amplitude peaks, it is difficult to separate the heartbeat amplitude peaks from the respiration amplitude peaks. Furthermore, if a harmonic frequency that is three or four times a respiration frequency is approximately equal to a heartbeat frequency, it is difficult to separate the heartbeat amplitude peaks from the respiration amplitude peaks. Even if the heartbeat amplitude peaks can be separated from the respiration amplitude peaks, problems described in Fig. 2 and Fig. 3 exist.

Fig. 2 illustrates a problem of heartbeat detection processing of the prior art. In one heartbeat, amplitude peaks of a first sound and a second sound are present. Accordingly, it is difficult to avoid a possibility that a heart rate (four times of (1), (2), (3), and (4)) which is twice a true heart rate (two times of (1) and (3) or (2) and (4)) is calculated. When A (bpm) is a detection object as a heart rate, 2A (bpm) cannot be a detection object as a heart rate, and therefore, it is difficult to extend a detection range of a heart rate.

Fig. 3 illustrates a problem of respiration detection processing of the prior art. Although, in one respiration, the movements of a chest and an abdomen are out of synchronization in some cases, the movements of the chest and the abdomen are synthesized without being discriminated in a radar signal. Accordingly, it is difficult to avoid a possibility that a respiration rate (four times of (1), (2), (3), and (4) of respiration, synthesis) which is twice a true respiration rate (two times of (1) and (2) of respiration, chest or respiration, abdomen) is calculated. When B (bpm) is a detection object as a respiration rate, 2B (bpm) cannot be a detection object as a respiration rate, and therefore, it is difficult to extend a detection range of a respiration rate.

Therefore, in order to solve the above-described problems, an object of this disclosure is to ensure the improvement of robustness, the reduction of the effects of disturbances, the separation of heartbeat and respiration, the avoidance of multiple times counting, and the extension of a detection range when calculating a heart rate and a respiration rate while enabling the alleviation of burdens on nurses and infection risk reduction based on a radar signal (an ultrasound signal may be included) reflecting off a body surface.

Solution to Problem

In order to solve the above-described problem of heartbeat detection, a frequency component caused by a micro vibration of heartbeat is extracted from a radar signal or an ultrasound signal reflecting off a body surface over a predefined heartbeat observation time window. Then, amplitude peaks are extracted from the frequency component caused by the micro vibration of the heartbeat, and one characteristic micro vibration out of a plurality of characteristic micro vibrations in one heartbeat is extracted.

Specifically, this disclosure is a heartbeat and respiration detection device including: a heartbeat component extraction unit that extracts a frequency component caused by a micro vibration of heartbeat from a radar signal or an ultrasound signal reflecting off a body surface over a predefined heartbeat observation time window; a heartbeat peak extraction unit that extracts an amplitude peak from the frequency component caused by the micro vibration of the heartbeat and extracts one characteristic micro vibration out of a plurality of characteristic micro vibrations in one heartbeat; and a heart rate calculation unit that calculates a heart rate based on a time interval of the amplitude peaks or a count of the amplitude peaks extracted within a predetermined time.

With this configuration, since the frequency component caused by the micro vibration of the heartbeat is extracted, the improvement of robustness and the reduction of the effects of disturbances can be ensured. Then, since one characteristic micro vibration out of the plurality of characteristic micro vibrations in one heartbeat is extracted, the avoidance of multiple times counting and the extension of a detection range can be ensured. Furthermore, since data that serves as a basis for calculating the heart rate is different from data that serves as a basis for calculating a respiration rate (described below), the separation of heartbeat and respiration can be ensured.

In addition, this disclosure is the heartbeat and respiration detection device in which the heartbeat component extraction unit extracts the frequency component caused by the micro vibration of the heartbeat over the predefined heartbeat observation time window including a plurality of characteristic micro vibrations in one heartbeat.

With this configuration, since the plurality of characteristic micro vibrations in one heartbeat are synthesized and output at the time of extracting the frequency component caused by the micro vibration of the heartbeat, the avoidance of multiple times counting can be approximately completely ensured. However, depending on a time width of the predefined heartbeat observation time window, only one characteristic micro vibration in one heartbeat may be included in the predefined heartbeat observation time window.

In addition, this disclosure is the heartbeat and respiration detection device in which the heartbeat peak extraction unit extracts a maximum amplitude peak of the amplitude peaks from the frequency component caused by the micro vibration of the heartbeat in a predefined peak detection time window.

With this configuration, even when only one characteristic micro vibration in one heartbeat is included in the predefined heartbeat observation time window, only the maximum amplitude peak is extracted in the predefined peak detection time window, and therefore, the avoidance of multiple times counting can be approximately completely ensured. However, depending on a time width of the predefined peak detection time window, an upper limit and a lower limit of the heart rate are narrowed to some extent.

In addition, this disclosure is the heartbeat and respiration detection device in which the heartbeat peak extraction unit moves the predefined peak detection time window so as to extract the maximum amplitude peak in a time domain excluding vicinities of both ends of the predefined peak detection time window.

Since in this configuration, one characteristic micro vibration in one heartbeat is made to avoid straddling an adjacent predefined peak detection time window, and therefore, the upper limit of the heart rate can be extended.

In addition, this disclosure is the heartbeat and respiration detection device in which the heart rate calculation unit calculates a heart rate based on a time interval of the maximum amplitude peaks extracted in an adjacent or non-adjacent predefined peak detection time window.

With this configuration, the lower limit of the heart rate can be extended considering that the plurality of characteristic micro vibrations in one heartbeat are included in predefined peak detection time windows that lie at intervals.

In addition, this disclosure is the heartbeat and respiration detection device in which the heart rate calculation unit calculates an average heart rate with increasing a weight of a time interval of the amplitude peaks as an amplitude value of the amplitude peak increases.

With this configuration, since the time interval of the heartbeat amplitude peaks is weighted according to the magnitudes of the amplitude values of the heartbeat amplitude peaks, the calculation of the heart rate can be performed with high accuracy.

In addition, this disclosure is the heartbeat and respiration detection device in which the heart rate calculation unit calculates a heart rate based on clustering of two-dimensional data constituted of a time interval of the amplitude peaks and a weight of the time interval with increasing the weight of the time interval of the amplitude peaks as an amplitude value of the amplitude peak increases.

With this configuration, since the weight of the time interval of the heartbeat amplitude peaks is calculated according to the magnitudes of the amplitude values of the heartbeat amplitude peaks, the calculation of the heart rate can be performed with high accuracy.

In order to solve the above-described problem of respiration detection, positive and negative frequency components caused by the micro vibration of the heartbeat is extracted from the radar signal or the ultrasound signal reflecting off the body surface, and complex multiplication of the positive and negative frequency components is performed. Then, from complex multiplication of the positive and negative frequency components, a respiration phase change caused by a micro vibration of respiration is extracted, with a heartbeat phase change caused by the micro vibration of the heartbeat removed.

Specifically, this disclosure is a heartbeat and respiration detection device including: a heartbeat component multiplication unit that extracts positive and negative frequency components caused by a micro vibration of heartbeat from a radar signal or an ultrasound signal reflecting off a body surface and performs complex multiplication of the positive and negative frequency components; a respiration phase extraction unit that extracts a respiration phase change caused by a micro vibration of respiration, with a heartbeat phase change caused by a micro vibration of heartbeat removed, from complex multiplication of the positive and negative frequency components; and a respiration rate calculation unit that calculates a respiration rate based on a frequency component of the respiration phase change.

With this configuration, since the frequency component caused by the micro vibration of the heartbeat is extracted, the improvement of robustness and the reduction of the effects of disturbances can be ensured. Then, since the respiration rate is calculated based on the frequency component of the respiration phase change with the heartbeat phase change removed, the avoidance of multiple times counting and the extension of a detection range can be ensured in consideration of a reflected signal from a chest without considering a reflected signal from an abdomen. Furthermore, since the data that serves as a basis for calculating the respiration rate is different from the data that serves as a basis for calculating the heart rate (described above), the separation of respiration and heartbeat can be ensured.

In addition, this disclosure is the heartbeat and respiration detection device in which the respiration phase extraction unit extracts amplitude peaks caused by a micro vibration of heartbeat from complex multiplication of the positive and negative frequency components and extracts the respiration phase change at the amplitude peak while applying zero padding between the amplitude peaks without extracting the respiration phase change.

With this configuration, in consideration of the fluctuation of the heartbeat, information on the respiration phase change is used at heartbeat amplitude peak time while the information on the respiration phase change is not used between the heartbeat amplitude peaks, and therefore, the calculation of the respiration rate can be performed with high accuracy.

In addition, this disclosure is the heartbeat and respiration detection device in which the respiration rate calculation unit calculates an average respiration rate with increasing a weight of a respiration rate as a maximum peak amplitude of the frequency component of the respiration phase change increases.

With this configuration, since the respiration rate is weighted according to the magnitude of the maximum peak amplitude of the frequency component of the respiration phase change, the calculation of the respiration rate can be performed with high accuracy.

In addition, this disclosure is a heartbeat and respiration detection program for causing a computer to execute each processing step corresponding to each processing unit of the heartbeat and respiration detection device described above.

This configuration allows providing the program having the above-described effects.

Advantageous Effects of Invention

Thus, this disclosure can ensure the improvement of robustness, the reduction of the effects of disturbances, the separation of heartbeat and respiration, the avoidance of multiple times counting, and the extension of a detection range when calculating a heart rate and a respiration rate while enabling the alleviation of burdens on nurses and infection risk reduction based on a radar signal (an ultrasound signal may be included) reflecting off a body surface.

BRIEF DESCRIPTION OF DRAWINGS

Fig. 1 is a drawing illustrating an outline of heartbeat and respiration detection processing of the prior art.
Fig. 2 is a drawing illustrating a problem of heartbeat detection processing of the prior art.
Fig. 3 is a drawing illustrating a problem of respiration detection processing of the prior art.
Fig. 4 is a diagram illustrating a configuration of a heartbeat and respiration detection device of this disclosure.
Fig. 5 is a drawing illustrating an outline of heartbeat and respiration detection processing of this disclosure.
Fig. 6 is a diagram illustrating a procedure of heartbeat detection processing of this disclosure.
Fig. 7 is a drawing illustrating a specific example of heartbeat component extraction processing of this disclosure.
Fig. 8 is a drawing illustrating a specific example of first heartbeat detection processing of this disclosure.
Fig. 9 is a drawing illustrating a specific example of the first heartbeat detection processing of this disclosure.
Fig. 10 is a drawing illustrating a specific example of the first heartbeat detection processing of this disclosure.
Fig. 11 is a drawing illustrating a specific example of second heartbeat detection processing of this disclosure.
Fig. 12 is a drawing illustrating a specific example of third heartbeat detection processing of this disclosure.
Fig. 13 is a drawing illustrating a specific example of the third heartbeat detection processing of this disclosure.
Fig. 14 is a diagram illustrating a procedure of respiration detection processing of this disclosure.
Fig. 15 is a drawing illustrating a principle of the respiration detection processing of this disclosure.
Fig. 16 is a drawing illustrating the principle of the respiration detection processing of this disclosure.
Fig. 17 is a drawing illustrating the principle of the respiration detection processing of this disclosure.

Fig. 18 is a drawing illustrating a specific example of the respiration detection processing of this disclosure.

Fig. 19 is a drawing illustrating a result of the heartbeat detection processing of this disclosure.

Fig. 20 is a drawing illustrating the result of the heartbeat detection processing of this disclosure.

Fig. 21 is a drawing illustrating a result of the respiration detection processing of this disclosure.

Fig. 22 is a drawing illustrating the result of the respiration detection processing of this disclosure.

Fig. 23 is a drawing illustrating the result of the respiration detection processing of this disclosure.

DESCRIPTION OF EMBODIMENTS

Embodiments of this disclosure will be described by referring to the accompanying drawings. The embodiments described below are examples of the implementation of this disclosure, and this disclosure is not limited to the following embodiments.

(Configuration of Heartbeat and Respiration Detection Device of This Disclosure)

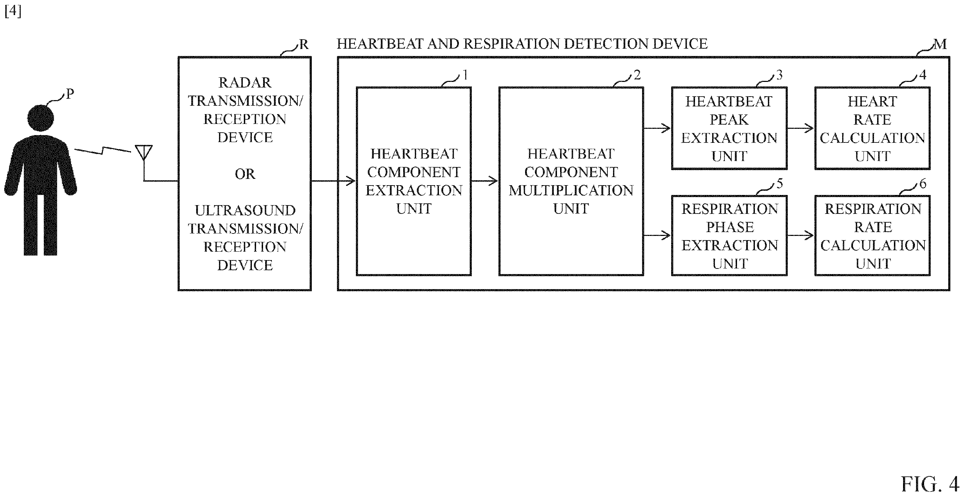

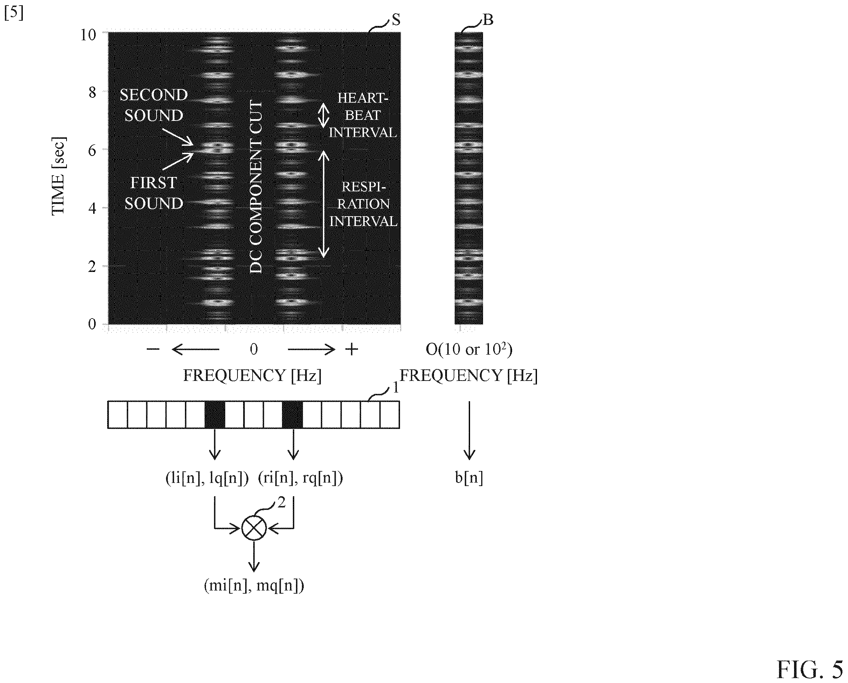

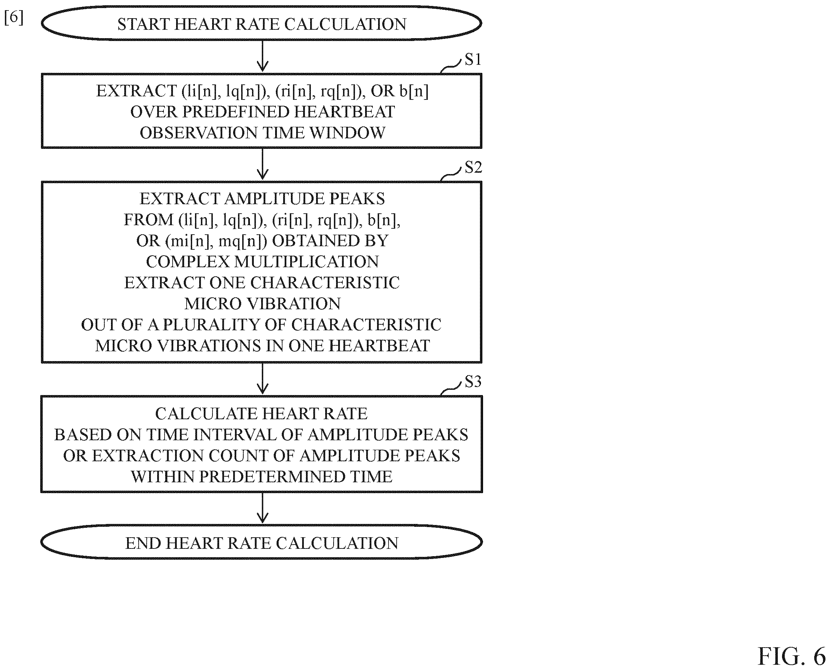

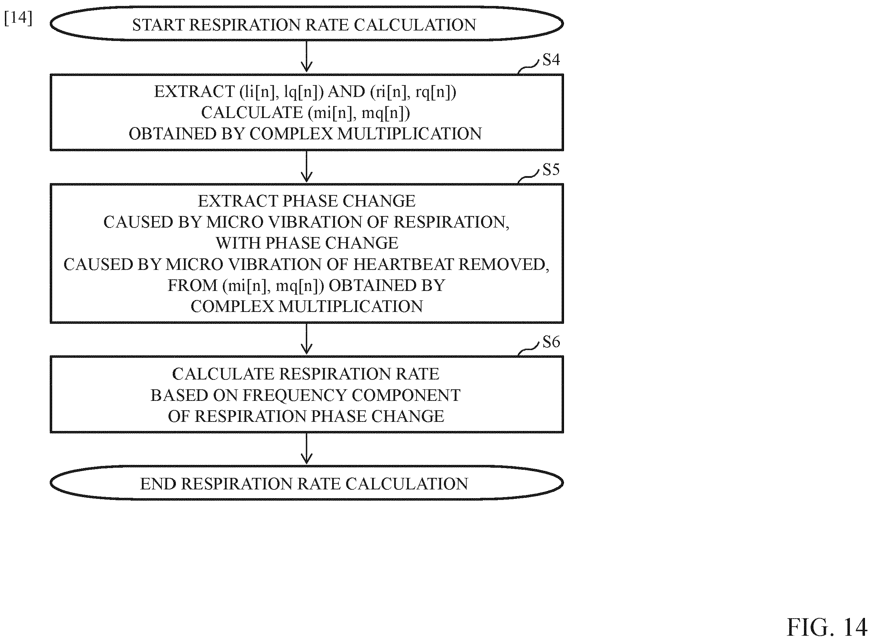

Fig. 4 illustrates a configuration of a heartbeat and respiration detection device of this disclosure. Fig. 5 illustrates an outline of heartbeat and respiration detection processing of this disclosure. A heartbeat and respiration detection device M is equipped with a heartbeat component extraction unit 1, a heartbeat component multiplication unit 2, a heartbeat peak extraction unit 3, a heart rate calculation unit 4, a respiration phase extraction unit 5, and a respiration rate calculation unit 6 and can be realized by installing a heartbeat and respiration detection program illustrated in Fig. 6 and Fig. 14 on a computer.

A radar transmission/reception device R or an ultrasound transmission/reception device R transmits a radar signal or an ultrasound signal (carrier wave band) with which a body surface of a patient P is irradiated, receives the radar signal or the ultrasound signal (carrier wave band) reflecting off the body surface of the patient P, and converts the received radar signal or ultrasound signal to a baseband to output it. A radar method or an ultrasound method may be any of a CW method, an FMCW method, a standing wave method, and other methods. The radar signal or the ultrasound signal (carrier wave band) has a wavelength on the order of 1 mm to 10 mm, which is equal to on the order of a micro vibration width of the body surface of the patient P.

The heartbeat component extraction unit 1 extracts one frequency component or both frequency components of positive and negative frequency components (on the order of $\pm 10$ or $10^2$ Hz) caused by a micro vibration of heartbeat from the radar signal or the ultrasound signal (I/Q complex signal of the baseband) reflecting off the body surface of the patient P. Alternatively, the heartbeat component extraction unit 1 extracts the frequency component (on the order of +10 or $10^2$ Hz) caused by the micro vibration of the heartbeat from the radar signal or the ultrasound signal (real number signal of the baseband) reflecting off the body surface of the patient P. The heartbeat component multiplication unit 2 performs complex multiplication of the positive and negative frequency components (on the order of $\pm 10$ or $10^2$ Hz) caused by the micro vibration of the heartbeat.

Here, the heartbeat component extraction unit 1 calculates a spectrogram S that shows a temporal change of each frequency component from the radar signal or the ultrasound signal (I/Q complex signal of the baseband) reflecting off the body surface of the patient P. Then, from the spectrogram S, a direct current (DC) component including an extremely low frequency component is removed, and the positive and negative frequency components (on the order of $\pm 10$ or $10^2$ Hz) caused by the micro vibration of the heartbeat are extracted. Alternatively, the heartbeat component extraction unit 1 calculates a band-pass filter result B in the frequency component caused by the micro vibration of the heartbeat from the radar signal or the ultrasound signal (real number signal of the baseband) reflecting off the body surface of the patient P. Then, in the band-pass filter result B, approximately similarly to the spectrogram S, the frequency component (on the order of +10 or $10^2$ Hz) caused by the micro vibration of the heartbeat is extracted.

In Fig. 5, about once or twice in 2 seconds, small amplitude peaks caused by the micro vibration of the heartbeat are extracted, and close amplitude peaks of a first sound and a second sound in one heartbeat are extracted. Then, although about once or twice in 4 seconds, large amplitude peaks caused by the micro vibration of the respiration are extracted, a harmonic frequency that is three or four times a respiration frequency may be approximately equal to a heartbeat frequency.

Here, the heartbeat component extraction unit 1 extracts (ri[n], rq[n]) (*r* is a positive frequency, *i* and *q* are *i* and *q* components, respectively, and *n* is time) as a positive frequency component caused by the micro vibration of the heartbeat and extracts (li[n], lq[n]) (*l* is a negative frequency, *i* and *q* are *i* and *q* components, respectively, and *n* is time) as a negative frequency component caused by the micro vibration of the heartbeat from the radar signal or the ultrasound signal (I/Q complex signal of the baseband) reflecting off the body surface of the patient P. Alternatively, the heartbeat component extraction unit 1 extracts b[n] (*b* is a pass frequency band of the band-pass filter result B, and *n* is time) as a frequency component caused by the micro vibration of the heartbeat from the radar signal or the ultrasound signal (real number signal of the baseband) reflecting off the body surface of the patient P. Then, the heartbeat component multiplication unit 2 calculates (mi[n], mq[n]) = (ri[n]·li[n] - rq[n]·lq[n], rq[n]·li[n] + ri[n]·lq[n]) as complex multiplication of the positive and negative frequency components.

As illustrated in Fig. 6 to Fig. 13, the heartbeat peak extraction unit 3 and the heart rate calculation unit 4 calculate a heart rate based on the frequency components (ri[n], rq[n]), (li[n], lq[n]), b[n], or (mi[n], mq[n]). As illustrated in Fig. 14 to Fig. 18, the respiration phase extraction unit 5 and the respiration rate calculation unit 6 calculate a respiration rate based on the frequency component (mi[n], mq[n]). First, the calculation of the heart rate is described, and next, the calculation of the respiration rate is described.

(Procedure of Heartbeat Detection Processing of This Disclosure: Heartbeat Component Extraction Processing)

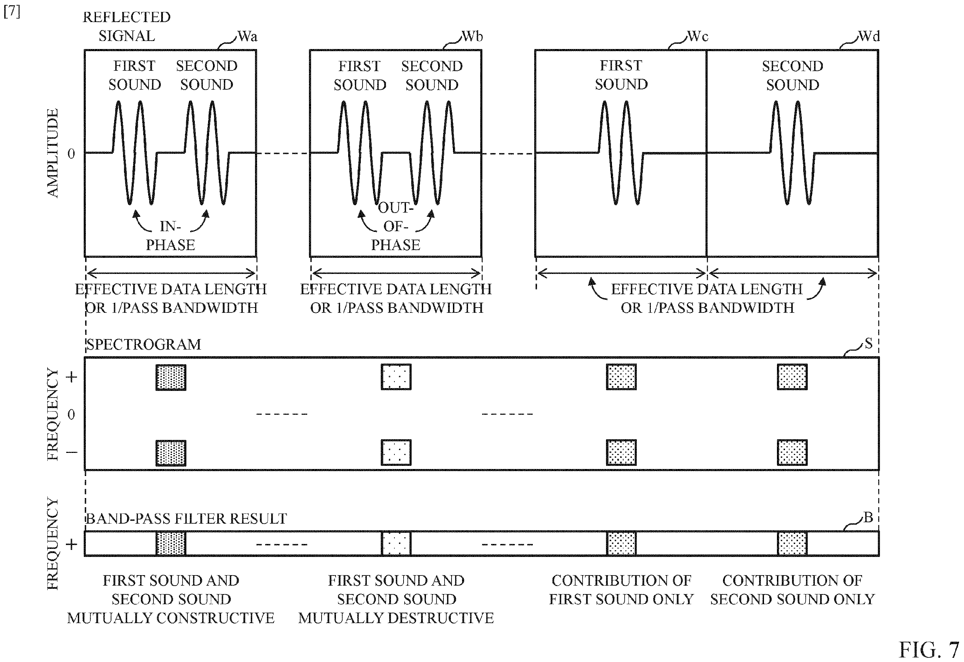

Fig. 6 illustrates a procedure of heartbeat detection processing of this disclosure. The heartbeat component extraction unit 1 extracts the frequency components (ri[n], rq[n]), (li[n], lq[n]), or b[n] over a predefined heartbeat observation time window (Step S 1). The heartbeat peak extraction unit 3 extracts amplitude peaks from the frequency components (ri[n], rq[n]), (li[n], lq[n]), b[n], or (mi[n], mq[n]) and extracts one characteristic micro vibration out of a plurality of characteristic micro vibrations in one heartbeat (Step S2). The heart rate calculation unit 4 calculates the heart rate based on a time interval of the amplitude peaks (this embodiment) or a count of the amplitude peaks extracted within a predetermined time (modification) (Step S3). Specifically, the processing from Fig. 7 to Fig. 13 is performed.

Thus, since the frequency component caused by the micro vibration of the heartbeat is extracted, the improvement of robustness and the reduction of the effects of disturbances can be ensured. Then, since one characteristic micro vibration out of the plurality of characteristic micro vibrations in one heartbeat is extracted, the avoidance of multiple times counting and the extension of a detection range can be ensured. Furthermore, since data that serves as a basis for calculating the heart rate is different from data that serves as a basis for calculating a respiration rate (described below), the separation of heartbeat and respiration can be ensured.

Here, in this embodiment, the first sound and the second sound in one heartbeat are employed as the plurality of characteristic micro vibrations in one heartbeat. However, as a modification, three or more characteristic micro vibrations in one heartbeat may be employed depending on an individual difference or animal species of a heartbeat and respiration detection object.

Fig. 7 illustrates a specific example of heartbeat component extraction processing of this disclosure. The heartbeat component extraction unit 1 extracts the frequency components (ri[n], rq[n]), (li[n], lq[n]), or b[n] over a predefined heartbeat observation time window including a plurality of characteristic micro vibrations in one heartbeat (Step S 1).

In Fig. 7, a time width of heartbeat observation time windows Wa, Wb, Wc, and Wd is an effective data length or 1/(pass bandwidth of the band-pass filter result B) of the spectrogram S.

In the heartbeat observation time window Wa, the first sound and the second sound in one heartbeat are included in an in-phase state (the phase relationship varies depending on individual differences or animal species). Then, in the spectrogram S, after the first sound and the second sound in one heartbeat are synthesized in a mutually constructive state, the frequency components (ri[n], rq[n]) and (li[n], lq[n]) are extracted. Alternatively, in the band-pass filter result B, after the first sound and the second sound in one heartbeat are synthesized in a mutually constructive state, the frequency component b[n] is extracted. Accordingly, at the time of extracting the frequency components caused by the micro vibration of the heartbeat, the avoidance of multiple times counting can be approximately completely ensured.

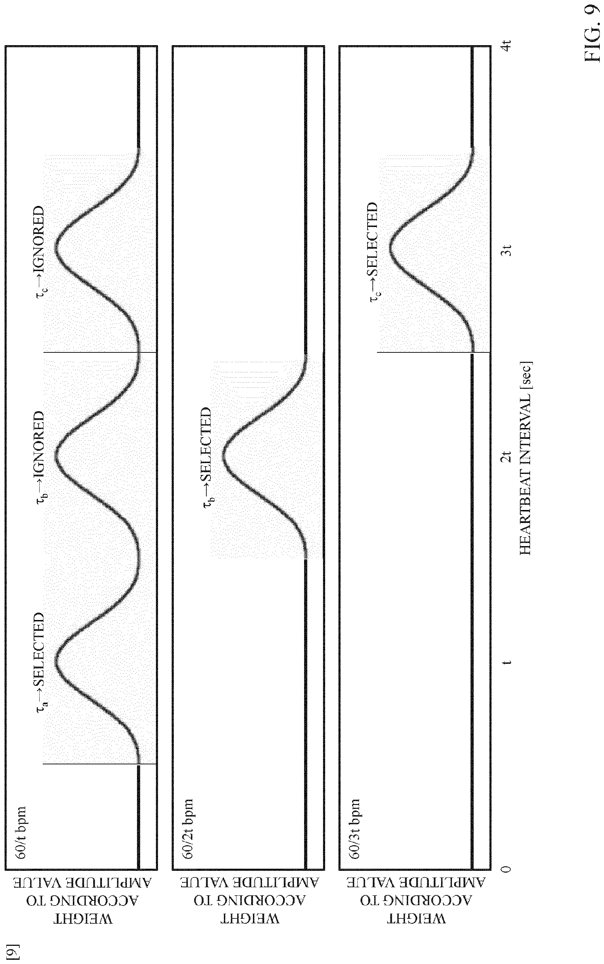

In the heartbeat observation time window Wb, the first sound and the second sound in one heartbeat are included in an out-of-phase state (the phase relationship varies depending on individual differences or animal species). Then, in the spectrogram S, after the first sound and the second sound in one heartbeat are synthesized in a mutually destructive state, the frequency components (ri[n], rq[n]) and (li[n], lq[n]) are extracted. Alternatively, in the band-pass filter result B, after the first sound and the second sound in one heartbeat are synthesized in a mutually destructive state, the frequency component b[n] is extracted. However, if $\tau_a$, $\tau_b$, $\tau_c$, and the like are considered as illustrated in Fig. 9, Fig. 10, and Fig. 13, the avoidance of multiple times counting can be approximately completely ensured.

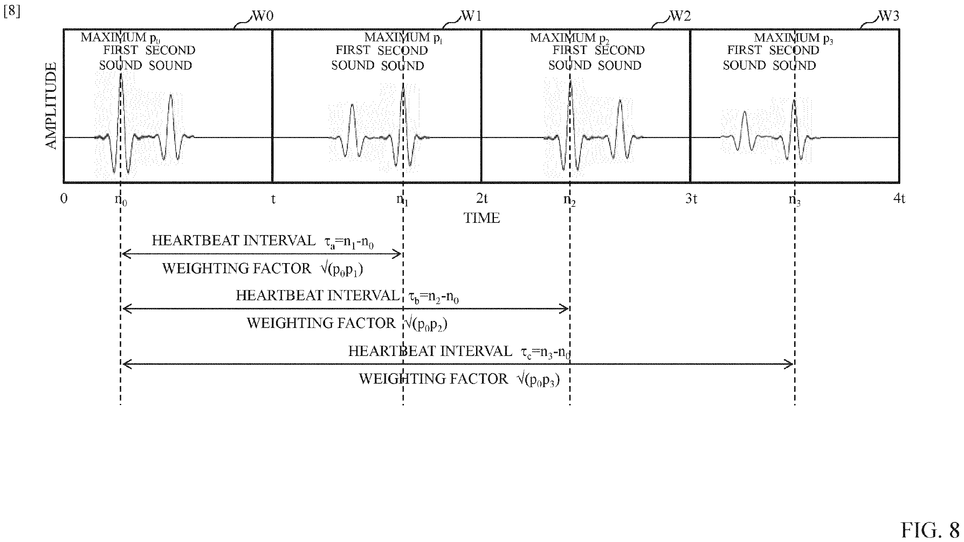

In the heartbeat observation time window Wc, only the first sound in one heartbeat is included (the number of micro vibrations varies depending on the situation of heartbeat and respiration). Then, in the spectrogram S, only for the first sound in one heartbeat, the frequency components (ri[n], rq[n]) and (li[n], lq[n]) are extracted. Alternatively, in the band-pass filter result B, only for the first sound in one heartbeat, the frequency component b[n] is extracted. However, if the peak detection time window is applied as illustrated in Fig. 8 to Fig. 13, the avoidance of multiple times counting can be approximately completely ensured.

In the heartbeat observation time window Wd, only the second sound in one heartbeat is included (the number of micro vibrations varies depending on the situation of heartbeat and respiration). Then, in the spectrogram S, only for the second sound in one heartbeat, the frequency components (ri[n], rq[n]) and (li[n], lq[n]) are extracted. Alternatively, in the band-pass filter result B, only for the second sound in one heartbeat, the frequency component b[n] is extracted. However, if the peak detection time window is applied as illustrated in Fig. 8 to Fig. 13, the avoidance of multiple times counting can be approximately completely ensured.

(Procedure of Heartbeat Detection Processing of This Disclosure: First Heartbeat Detection Processing)

Fig. 8 to Fig. 10 illustrate specific examples of first heartbeat detection processing of this disclosure. The heartbeat peak extraction unit 3 extracts maximum amplitude peaks of the amplitude peaks from the frequency components (ri[n], rq[n]), (li[n], lq[n]), b[n], or (mi[n], mq[n]) within predefined peak detection time windows WO, W1, W2, and W3 (Step S2).

In Fig. 8, the amplitude peaks of the first sound and the second sound in one heartbeat are present within the peak detection time windows WO, W1, W2, and W3 with a predefined time width $t$ (a peak detection time window is slightly longer than a heartbeat observation time window). Within the peak detection time window WO, the amplitude peak of the first sound has the peak time $n_0$ and a maximum amplitude value $p_0$. Within the peak detection time window W1, the amplitude peak of the second sound has the peak time $n_1$ and a maximum amplitude value $p_1$. Within the peak detection time window W2, the amplitude peak of the first sound has the peak time $n_2$ and a maximum amplitude value $p_2$. Within the peak detection time window W3, the amplitude peak of the second sound has the peak time $n_3$ and a maximum amplitude value $p_3$.

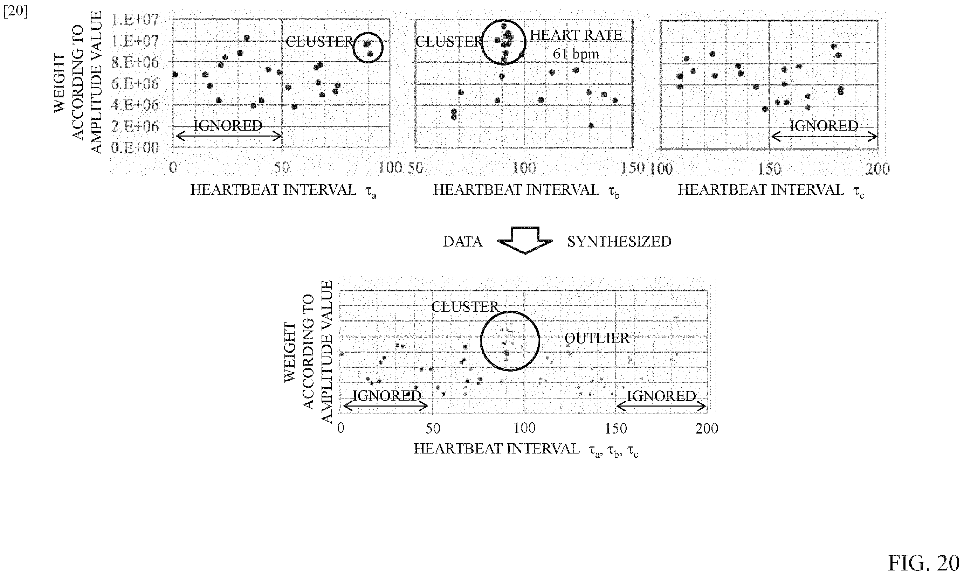

The heart rate calculation unit 4 calculates an average heart rate with increasing a weight of the time interval of the amplitude peaks as amplitude values of the amplitude peaks increase (Step S3). Alternatively, the heart rate calculation unit 4 calculates the heart rate based on clustering of two-dimensional data constituted of the time interval of the amplitude peaks and the weight of the time interval with increasing the weight of the time interval of the amplitude peaks as the amplitude values of the amplitude peaks increase (Step S3, Fig. 20).

The heart rate calculation unit 4 calculates the heart rate based on the time interval of the maximum amplitude peaks extracted in adjacent or non-adjacent predefined peak detection time windows (Step S3). In Fig. 8, between the peak detection time windows WO and W1, a heartbeat interval $\tau_a$ is $n_1$-$n_0$ and has a weighting factor of $\sqrt{(p_0 p_1)}$. Between the peak detection time windows WO and W2, a heartbeat interval $\tau_b$ is $n_2$-$n_0$ and has a weighting factor of $\sqrt{(p_0 p_2)}$. Between the peak detection time windows WO and W3, a heartbeat interval $\tau_c$ is $n_3$-$n_0$ and has a weighting factor of $\sqrt{(p_0 p_3)}$.

In Fig. 9, average heartbeat intervals $\tau_{a,ave}$, $\tau_{b,ave}$, and $\tau_{c,ave}$ are calculated as Math. 1 to Math. 3. Here, when a peak detection time window Wm (m = 0 to M-1) is used as a reference, $\tau_a = \tau_a[m]$, $\tau_b = \tau_b[m]$, $\tau_c = \tau_c[m]$, $p_0 = p_0[m]$, $p_1 = p_1[m]$, $p_2 = p_2[m]$, and $p_3 = p_3[m]$. Then, the average heartbeat intervals $\tau_{a,ave}$, $\tau_{b,ave}$, and $\tau_{c,ave}$ are average values from a start (m = 0) to an end (m = M-1) of a detection period of the heart rate.

[Math. 1]

$$\overline{\tau_a} = \frac{\sum_{m=0}^{M-1} \tau_a[m] \cdot \sqrt{p_1[m] \cdot p_0[m]}}{\sum_{m=0}^{M-1} \sqrt{p_1[m] \cdot p_0[m]}}$$

[Math. 2]

$$\overline{\tau_b} = \frac{\sum_{m=0}^{M-1} \tau_b[m] \cdot \sqrt{p_2[m] \cdot p_0[m]}}{\sum_{m=0}^{M-1} \sqrt{p_2[m] \cdot p_0[m]}}$$

[Math. 3]

$$\overline{\tau_c} = \frac{\sum_{m=0}^{M-1} \tau_c[m] \cdot \sqrt{p_3[m] \cdot p_0[m]}}{\sum_{m=0}^{M-1} \sqrt{p_3[m] \cdot p_0[m]}}$$

When the average heart rate is 60/3t (bpm), a weight of the heartbeat interval $\tau_c$ has a peak in the average heartbeat interval $\tau_{c,ave}$, and the average heartbeat interval $\tau_{c,ave}$ is selected. When the average heart rate is 60/2t (bpm), a weight of the heartbeat interval $\tau_b$ has a peak in the average heartbeat interval $\tau_{b,ave}$, and the average heartbeat interval $\tau_{b,ave}$ is selected. When the average heart rate is 60/t (bpm), weights of the heartbeat intervals $\tau_a$, $\tau_b$, and $\tau_c$ have peaks in the average heartbeat intervals $\tau_{a,ave}$, $\tau_{b,ave}$, and $\tau_{c,ave}$, and only the average heartbeat interval $\tau_{a,ave}$ is selected. As illustrated in the lower stage of Fig. 20 described below, synthetic two-dimensional data may be created.

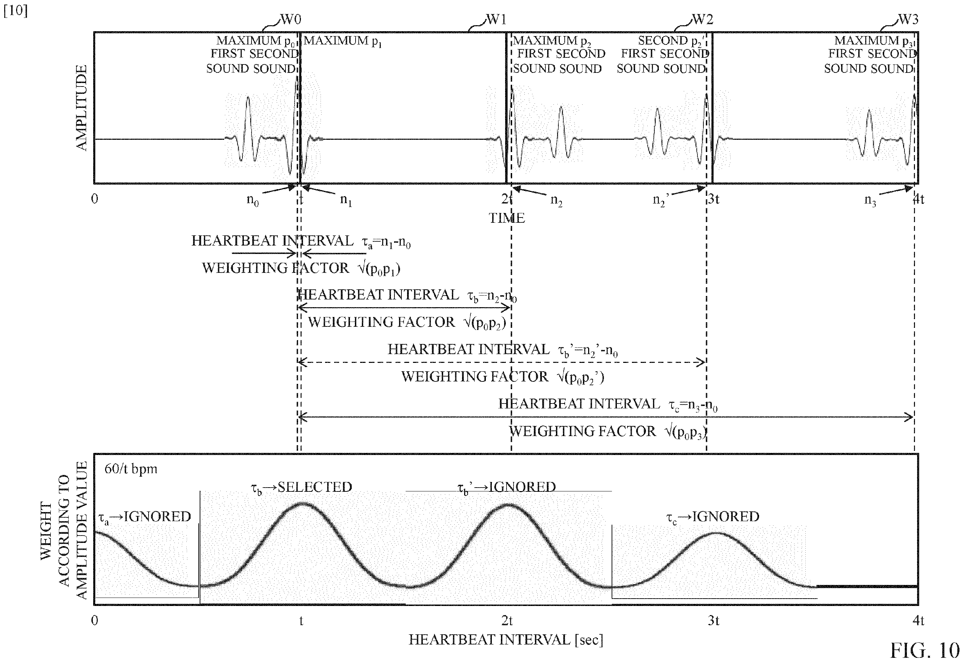

In Fig. 10, the amplitude peaks of the first sound and the second sound in one heartbeat are present at boundaries of the peak detection time windows WO, W1, W2, and W3 with the predefined time width $t$ (a peak detection time window is slightly longer than a heartbeat observation time window). Within the peak detection time window WO, the amplitude peak of the second sound has the peak time $n_0$ and the maximum amplitude value $p_0$. Within the peak detection time window W1, the amplitude peak of the second sound has the peak time $n_1$ (= $t$) and the maximum amplitude value $p_1$. Within the peak detection time window W2, the amplitude peak of the first sound for the first time has the peak time $n_2$ and the maximum amplitude value $p_2$, and the amplitude peak of the second sound for the second time has the peak time $n_2$' and a second amplitude value $p_2$'. Within the peak detection time window W3, the amplitude peak of the second sound has the peak time $n_3$ and the maximum amplitude value $p_3$.

In Fig. 10, between the peak detection time windows WO and W1, the heartbeat interval $\tau_a$ is $n_1$-$n_0$ and has a weighting factor of $\sqrt{(p_0 p_1)}$ (since the heartbeat interval $\tau_a$ is a short time, it is ignored as described below). Between the peak detection time windows WO and W2, the heartbeat interval $\tau_b$ is $n_2 - n_0$ and has a weighting factor of $\sqrt{(p_0 p_2)}$, and a heartbeat interval $\tau_b'$ is $n_2' - n_0$ and has a weighting factor of $\sqrt{(p_0 p_2')}$. Between the peak detection time windows W0 and W3, the heartbeat interval $\tau_c$ is $n_3 - n_0$ and has a weighting factor of $\sqrt{(p_0 p_3)}$.

In Fig. 10, average heartbeat intervals $\tau_{a,ave} = \Sigma \tau_a \sqrt{(p_0 p_1)}/\Sigma \sqrt{(p_0 p_1)}$, $\tau_{b,ave} = \Sigma \tau_b \sqrt{(p_0 p_2)}/\Sigma \sqrt{(p_0 p_2)}$, $\tau_{b,ave}' = \Sigma \tau_b' \sqrt{(p_0 p_2')}/\Sigma \sqrt{(p_0 p_2')}$, and $\tau_{c,ave} = \Sigma \tau_c \sqrt{(p_0 p_3)}/\Sigma \sqrt{(p_0 p_3)}$, are calculated (the sums are obtained over the detection period of the heart rate). When the average heart rate is $60/t$ (bpm), weights of the heartbeat intervals $\tau_a$, $\tau_b$, $\tau_b'$, and $\tau_c$ have peaks in the average heartbeat intervals $\tau_{a,ave}$, $\tau_{b,ave}$, $\tau_{b,ave}'$, and $\tau_{c,ave}$, and only the average heartbeat interval $\tau_{b,ave}$ is selected. As illustrated in the lower stage of Fig. 20 described below, synthetic two-dimensional data may be created.

Thus, even when only one characteristic micro vibration in one heartbeat is included in a predefined heartbeat observation time window, only the maximum amplitude peak is extracted in a predefined peak detection time window, and therefore, the avoidance of multiple times counting can be approximately completely ensured. However, depending on a time width of the predefined peak detection time window, an upper limit $60/t$ (bpm) and a lower limit $60/3t$ (bpm) of the heart rate are narrowed to some extent. Then, since the time interval of the heartbeat amplitude peaks is weighted according to the magnitudes of the amplitude values of the heartbeat amplitude peaks, the calculation of the heart rate can be performed with high accuracy. Furthermore, since the weight of the time interval of the heartbeat amplitude peaks is calculated according to the magnitudes of the amplitude values of the heartbeat amplitude peaks, the calculation of the heart rate can be performed with high accuracy.

(Procedure of Heartbeat Detection Processing of This Disclosure: Second Heartbeat Detection Processing)

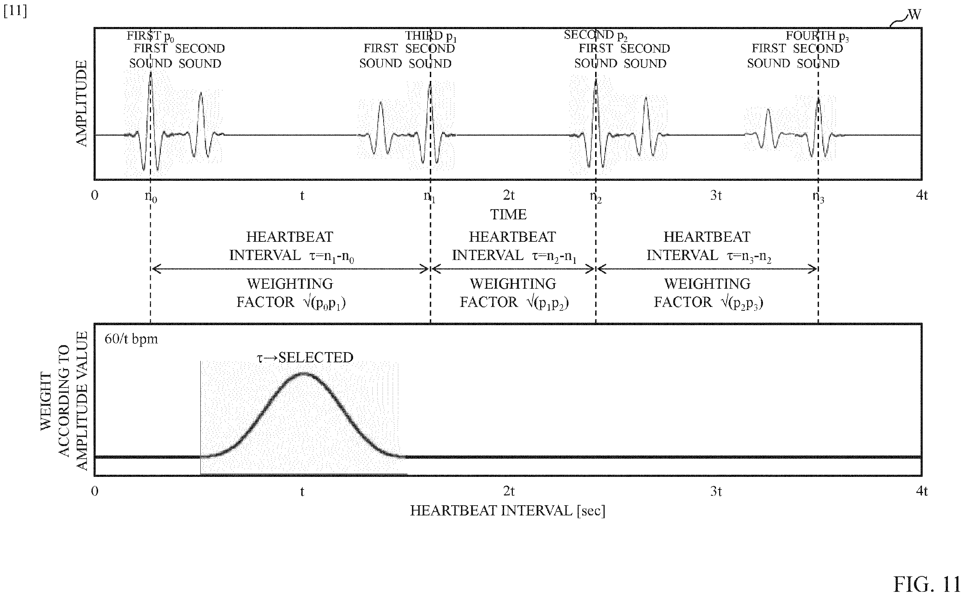

Fig. 11 illustrates a specific example of second heartbeat detection processing of this disclosure. The heartbeat peak extraction unit 3 extracts amplitude peaks within a predefined number or with a predefined amplitude or more from the frequency components $(ri[n], rq[n])$, $(li[n], lq[n])$, $b[n]$, or $(mi[n], mq[n])$ in a predefined peak detection time window W, which is longer than the predefined peak detection time windows W0 to W3 (Step S2).

In Fig. 11, the amplitude peaks of the first sound and the second sound in four heartbeats are present in the peak detection time window W (the time width is $4t$). In chronological order in the peak detection time window W (the time width is $4t$), the amplitude peak of the first sound has the peak time $n_0$ and a first amplitude value $p_0$, the amplitude peak of the second sound has the peak time $n_1$ and a third amplitude value $p_1$ the amplitude peak of the first sound has the peak time $n_2$ and a second amplitude value $p_2$, and the amplitude peak of the second sound has the peak time $n_3$ and a fourth amplitude value $p_3$. A known QRS detection algorithm, such as Engelse and Zeelenberg, may be used to extract the amplitude peaks within a predefined number or with a predefined amplitude or more.

The heart rate calculation unit 4 calculates an average heart rate with increasing a weight of the time interval of the amplitude peaks as the amplitude values of the amplitude peaks increase (Step S3). Alternatively, the heart rate calculation unit 4 calculates the heart rate based on clustering of two-dimensional data constituted of the time interval of the amplitude peaks and the weight of the time interval with increasing the weight of the time interval of the amplitude peaks as the amplitude values of the amplitude peaks increase (Step S3, Fig. 20).

In Fig. 11, in chronological order in the peak detection time window W (the time width is $4t$), the first adjacent heartbeat interval $\tau$ is $n_1 - n_0$ and has a weighting factor of $\sqrt{(p_0 p_1)}$, the second adjacent heartbeat interval $\tau$ is $n_2 - n_1$ and has a weighting factor of $\sqrt{(p_1 p_2)}$, and the third adjacent heartbeat interval $\tau$ is $n_3 - n_2$ and has a weighting factor of $\sqrt{(p_2 p_3)}$.

In Fig. 11, an average heartbeat interval $\tau_{ave} = \Sigma \tau \sqrt{(p_n p_{n+1})}/\Sigma \sqrt{(p_n p_{n+1})}$ is calculated (the sum is obtained over the detection period of the heart rate). When the average heart rate is $60/t$ (bpm), the weight of the heartbeat interval $\tau$ has a peak in the average heartbeat interval $\tau_{ave}$. As illustrated in the lower stage of Fig. 20 described below, synthetic two-dimensional data may be created.

Thus, even when only one characteristic micro vibration in one heartbeat is included in a predefined heartbeat observation time window, amplitude peaks within a predefined number or with a predefined amplitude or more are extracted in a predefined peak detection time window, and therefore, an upper limit and a lower limit of the heart rate can be extended to a sufficient degree compared with Fig. 8 to Fig. 10. However, depending on the predefined number and the predefined amplitude, the occurrence of multiple times counting is brought about to some extent. Then, since the time interval of the heartbeat amplitude peaks is weighted according to the magnitudes of the amplitude values of the heartbeat amplitude peaks, the calculation of the heart rate can be performed with high accuracy. Furthermore, since the weight of the time interval of the heartbeat amplitude peaks is calculated according to the magnitudes of the amplitude values of the heartbeat amplitude peaks, the calculation of the heart rate can be performed with high accuracy.

(Procedure of Heartbeat Detection Processing of This Disclosure: Third Heartbeat Detection Processing)

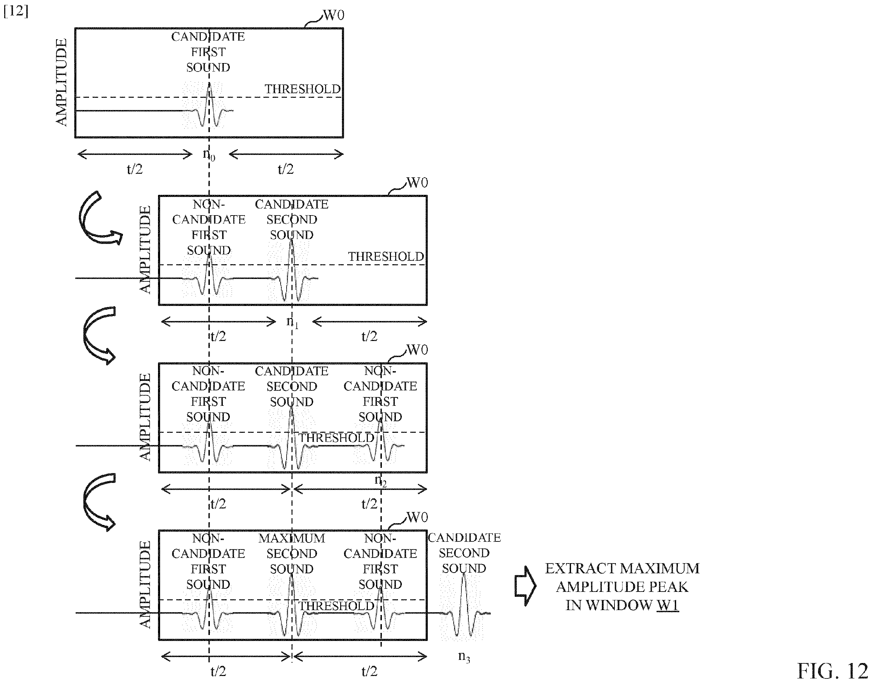

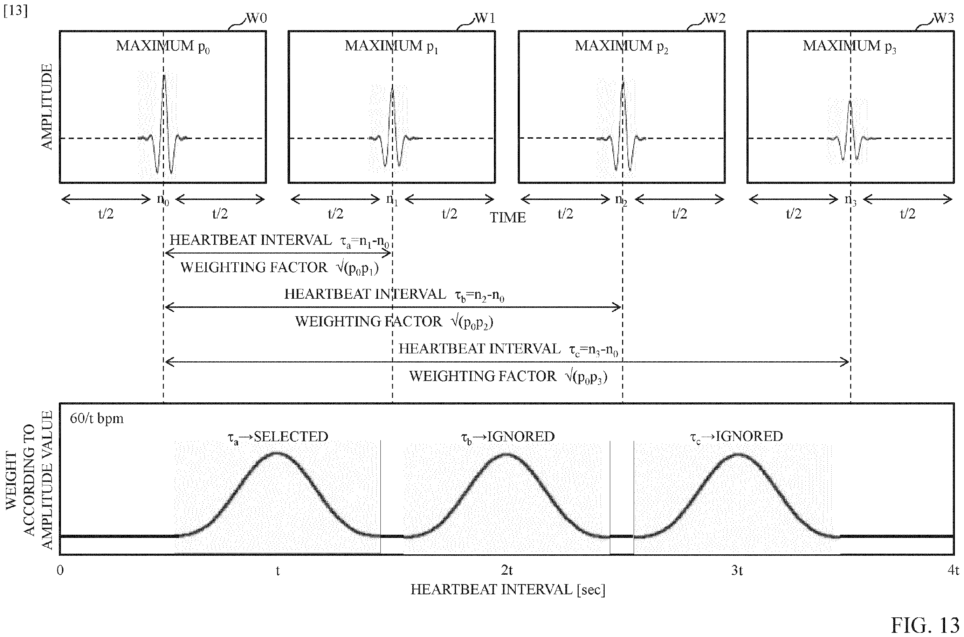

Fig. 12 and Fig. 13 illustrate specific examples of third heartbeat detection processing of this disclosure. The heartbeat peak extraction unit 3 moves the predefined peak detection time windows W0, W1, W2, and

W3 so as to extract the maximum amplitude peaks in time domains excluding vicinities of both ends of the predefined peak detection time windows WO, W1, W2, and W3 (Step S2). This is for not causing the short-time heartbeat interval $\tau_a$ of Fig. 10.

In Fig. 12, the peak detection time window WO with the predefined time width $t$ is moved according to the amplitude peaks of the first sound and the second sound in one heartbeat (a peak detection time window is slightly longer than a heartbeat observation time window). In the first stage of Fig. 12, an amplitude peak of a first sound has an amplitude value greater than a predefined noise threshold and is selected as a candidate for the maximum amplitude peak. Then, the peak detection time window WO is set such that time $n_0$ of the candidate for the maximum amplitude peak is located at the central time of the peak detection time window WO.

In the second stage of Fig. 12, an amplitude peak of a second sound has an amplitude value greater than the predefined noise threshold, has the amplitude value greater than the previous candidate for the maximum amplitude peak, and is selected as a new candidate for the maximum amplitude peak. Then, the peak detection time window WO is moved by an amount of time $n_1-n_0$ such that time $n_1$ of the new candidate for the maximum amplitude peak is located at the central time of the peak detection time window WO.

In the third stage of Fig. 12, an amplitude peak of a first sound has an amplitude value greater than the predefined noise threshold, has the amplitude values smaller than the previous candidate for the maximum amplitude peak, and is not selected as a new candidate for the maximum amplitude peak. Then, the peak detection time window WO is fixed with no change such that the time $n_1$ of the previous candidate for the maximum amplitude peak is located at the central time of the peak detection time window WO.

In the fourth stage of Fig. 12, an amplitude peak of a second sound has an amplitude value greater than the predefined noise threshold but is not included in the previous peak detection time window WO. Therefore, the new peak detection time window W1 is moved according to the amplitude peaks of the first sound and the second sound in one heartbeat. That is, the amplitude peak of the second sound has an amplitude value greater than the predefined noise threshold and is selected as a candidate for the maximum amplitude peak. Then, the peak detection time window W1 is set such that time $n_3$ of the candidate for the maximum amplitude peak is located at the central time of the peak detection time window W1. Subsequently, similar processing is repeated.

The heart rate calculation unit 4 calculates an average heart rate with increasing a weight of the time interval of the amplitude peaks as the amplitude values of the amplitude peaks increase (Step S3). Alternatively, the heart rate calculation unit 4 calculates the heart rate based on clustering of two-dimensional data constituted of the time interval of the amplitude peaks and the weight of the time interval with increasing the weight of the time interval of the amplitude peaks as the amplitude values of the amplitude peaks increase (Step S3, Fig. 20).

The heart rate calculation unit 4 calculates the heart rate based on the time interval of the maximum amplitude peaks extracted in adjacent or non-adjacent predefined peak detection time windows (Step S3). In Fig. 13, between the peak detection time windows WO and W1, the heartbeat interval $\tau_a$ is $n_1-n_0$ and has a weighting factor of $\sqrt{(p_0 p_1)}$. Between the peak detection time windows WO and W2, the heartbeat interval $\tau_b$ is $n_2-n_0$ and has a weighting factor of $\sqrt{(p_0 p_2)}$. Between the peak detection time windows WO and W3, the heartbeat interval $\tau_c$ is $n_3-n_0$ and has a weighting factor of $\sqrt{(p_0 p_3)}$.

In Fig. 13, the average heartbeat intervals $\tau_{a,ave} = \Sigma\tau_a\sqrt{(p_0 p_1)}/\Sigma\sqrt{(p_0 p_1)}$, $\tau_{b,ave} = \Sigma\tau_b\sqrt{(p_0 p_2)}/\Sigma\sqrt{(p_0 p_2)}$, and $\tau_{c,ave} = \Sigma\tau_c\sqrt{(p_0 p_3)}/\Sigma\sqrt{(p_0 p_3)}$ are calculated (the sums are obtained over the detection period of the heart rate). When the average heart rate is 60/t (bpm), the weights of the heartbeat intervals $\tau_a$, $\tau_b$, and $\tau_c$ have peaks in the average heartbeat intervals $\tau_{a,ave}$, $\tau_{b,ave}$, and $\tau_{c,ave}$, and only the average heartbeat interval $\tau_{a,ave}$ is selected. Alternatively, as illustrated in the lower stage of Fig. 20 described below, synthetic two-dimensional data may be created.

Thus, since one characteristic micro vibration in one heartbeat is made to avoid straddling an adjacent predefined peak detection time window, and therefore, the upper limit of the heart rate can be extended without causing the short-time heartbeat interval $\tau_a$ of Fig. 10. Meanwhile, when one characteristic micro vibration in one heartbeat is smaller than the predefined noise threshold, it is not selected as a candidate for the maximum amplitude peak, and therefore, the lower limit of the heart rate can be extended. Then, since the time interval of the heartbeat amplitude peaks is weighted according to the magnitudes of the amplitude values of the heartbeat amplitude peaks, the calculation of the heart rate can be performed with high accuracy. Furthermore, since the weight of the time interval of the heartbeat amplitude peaks is calculated according to the magnitudes of the amplitude values of the heartbeat amplitude peaks, the calculation of the heart rate can be performed with high accuracy.

In the first to third heartbeat detection processing, even when $\tau_{ave} = \Sigma\tau\sqrt{(p_n p_{n+1})}/\Sigma\sqrt{(p_n p_{n+1})}$ is calculated as an average heartbeat interval, it is not necessary to prepare all class values of $\tau$. Then, after the weight of the time interval of the amplitude peaks is calculated, a weighted average of the two-dimensional data constituted of the time interval of the amplitude peaks and the weight of the time interval is calculated. Accordingly, the detection period of the heart rate can be shortened. In addition, when $\tau_n \leftarrow (1-\lambda)\tau_n + \lambda\tau_{n-1}$ is calculated as an average heartbeat interval, it is only necessary to set a forgetting coefficient $\lambda$ according to $\sqrt{(p_{n-1} p_n)}$, and compared with a case when the weighted average of the two-dimensional data in the detection period of the heart rate is calculated, burdens of calculating $\tau_n$ can be alleviated. Note that as the weighting factor, in addition to setting $\sqrt{(p_{n-1}p_n)}$, $\max(p_{n-1}, p_n)$ may be set, or $\min(p_{n-1}, p_n)$ may be set.

Here, when $\tau_n \leftarrow (1-\lambda)\tau_n + \lambda_{n-1}$ is calculated as an average heartbeat interval, making the forgetting coefficient $\lambda$ small (large) makes it easy (hard) to remove noise but hard (easy) to shorten time for convergence. Then, when a first-order IIR filter is applied, the filter can be simplified compared with a case when a moving average filter and an FIR filter are applied.

In this embodiment, a heart rate of $60/\tau$ (bpm) is calculated with high accuracy based on the time interval $\tau$ (seconds) of the amplitude peaks. As a modification, a heart rate of 60k/To (bpm) may be calculated more simply based on a count **k** (pieces) of the amplitude peaks extracted within a predetermined time To (seconds). For example, a heart rate of 60 bpm can be calculated by extracting only 10 amplitude peaks in a measurement for 10 seconds. However, if one more amplitude peak is erroneously extracted in the measurement for 10 seconds, a heart rate of 66 bpm is calculated, and an error of +10% occurs. Accordingly, the accuracy is higher when the time interval of the amplitude peaks is used, and a calculation amount is fewer when the count of the amplitude peaks extracted is used.

(Procedure of Respiration Detection Processing of This Disclosure)

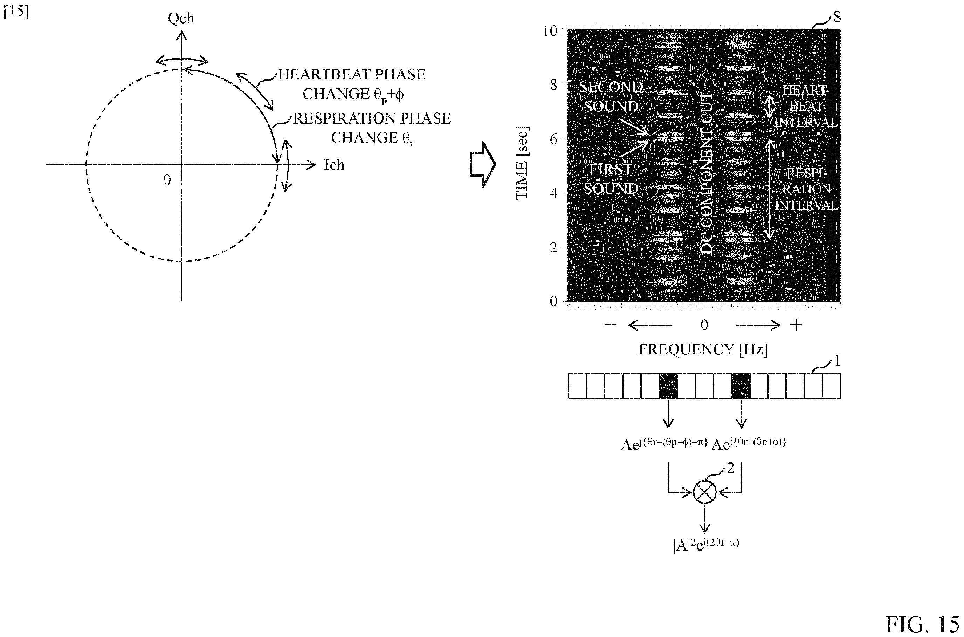

Fig. 14 illustrates a procedure of respiration detection processing of this disclosure. The heartbeat component extraction unit 1 extracts the frequency components (ri[n], rq[n]) and (li[n], lq[n]), and the heartbeat component multiplication unit 2 calculates the frequency component (mi[n], mq[n]) (Step S4). The respiration phase extraction unit 5 extracts a respiration phase change caused by the micro vibration of the respiration, with a heartbeat phase change caused by the micro vibration of the heartbeat removed, from the frequency component (mi[n], mq[n]) (Step S5). The respiration rate calculation unit 6 calculates the respiration rate based on a frequency component (for example, a Fourier transform component) of the respiration phase change (Step S6). Specifically, the processing from Fig. 15 to Fig. 18 is performed.

Fig. 15 to Fig. 17 illustrate a principle of the respiration detection processing of this disclosure. In Fig. 15, on the radar signal or the ultrasound signal (carrier wave band) reflecting off the body surface of the patient P, heartbeat phase modulation caused by the micro vibration of the heartbeat is performed, and respiration phase modulation caused by the micro vibration of the respiration is also performed. Here, on the radar signal or the ultrasound signal (carrier wave band) reflecting off the body surface of the patient P, although heartbeat amplitude modulation is also performed, the heartbeat amplitude modulation is uncertain compared with the heartbeat phase modulation and therefore disadvantageous for highly accurate respiration extraction, and although respiration amplitude modulation is also performed, the respiration amplitude modulation is uncertain compared with the respiration phase modulation and therefore disadvantageous for highly accurate respiration extraction.

Then, the heartbeat component extraction unit 1 extracts $Ae^{j\{\theta r+(\theta p+\phi)\}}$ (A is an amplitude, $\theta_r$ is a respiration phase change, $\theta_p$ is a heartbeat phase change, and $\phi$ is an initial phase of the heartbeat phase change) as a positive frequency component caused by the micro vibration of the heartbeat and extracts $Ae^{j\{\theta r-(\theta p+\phi)-\pi\}}$ ($\pi$ is a phase difference between upper and lower sideband waves of phase modulation) as a negative frequency component caused by the micro vibration of the heartbeat. Then, the heartbeat component multiplication unit 2 calculates $Ae^{j\{\theta r+(\theta p+\phi)\}}*Ae^{j\{\theta r-(\theta p+\phi)-\pi\}} = |A|^2 e^{j(2\theta r-\pi)}$ as complex multiplication of the positive and negative frequency components. Accordingly, from complex multiplication of the positive and negative frequency components $|A|^2 e^{j(2\theta r-\pi)}$, the respiration phase extraction unit 5 can extract the respiration phase change $\theta_r$ caused by the micro vibration of the respiration, with the heartbeat phase change $\theta_p+\phi$ caused by the micro vibration of the heartbeat removed.

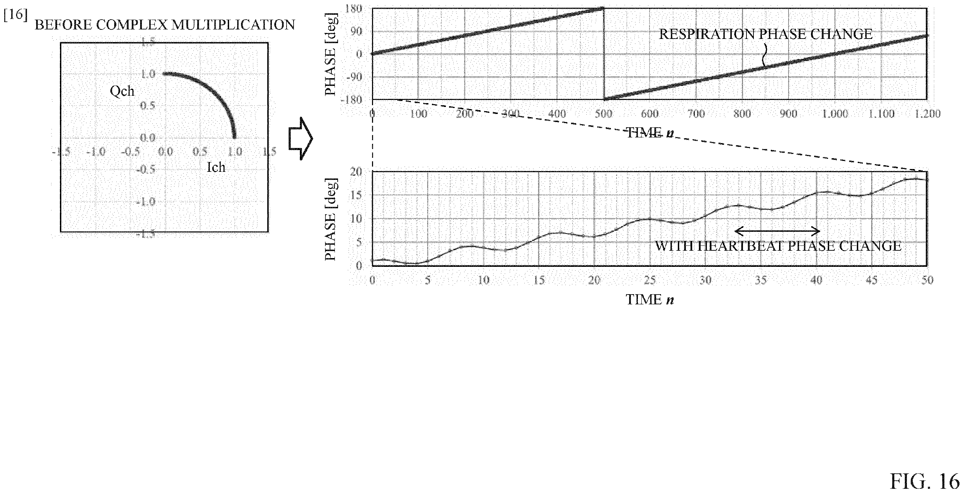

Fig. 16 illustrates a simulation result at a stage before the heartbeat component multiplication unit 2 calculates $|A|^2 e^{j(2\theta r-\pi)}$ as complex multiplication of the positive and negative frequency components. In the left section of Fig. 16, while the respiration phase change $\theta_r$ changes from 0 to $\pi/2$, the heartbeat phase change $\theta_p+\phi$ superimposes minute changes. In the upper right section of Fig. 16, a state where the respiration phase change $\theta_r$ changes is mainly observed, and in the lower right section of Fig. 16 (a part of the time domain is enlarged), a state where the respiration phase change $\theta_r$ changes is observed, and a state where the heartbeat phase change $\theta_p+\phi$ changes is also largely observed.

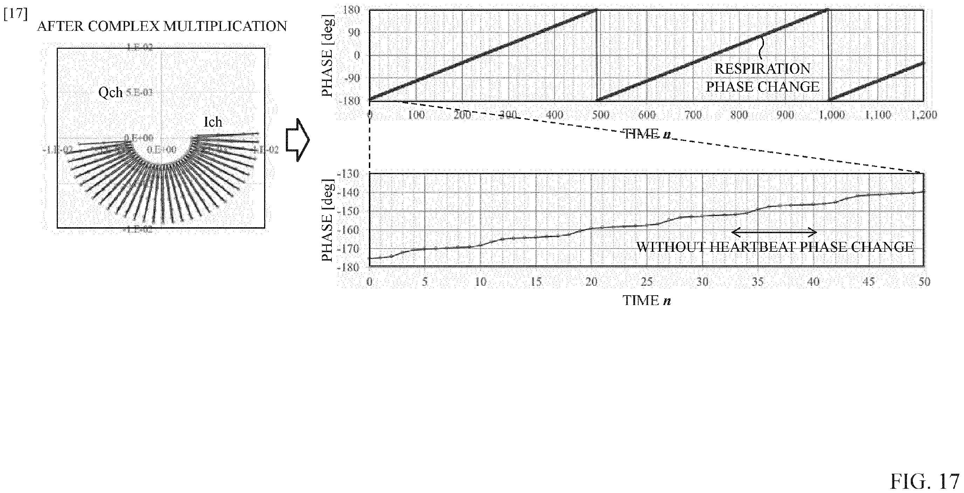

Fig. 17 illustrates a simulation result at a stage after the heartbeat component multiplication unit 2 calculates $|A|^2 e^{j(2\theta r-\pi)}$ as complex multiplication of the positive and negative frequency components. In the left section of Fig. 17, while a respiration phase change $2\theta_r-\pi$ changes from $-\pi$ to 0, the heartbeat phase change $\theta_p+\phi$ does not superimpose minute changes (although a change in amplitude is observed, a change in phase is a monotonous change). In the upper right section of Fig. 17, a state where the respiration phase change $2\theta_r-\pi$ changes is mainly observed, and in the lower right section of Fig. 17 (a part of the time domain is enlarged), although a state where the respiration phase change $2\theta_r-\pi$ changes is observed, a state where the heartbeat phase change $\theta_p+\phi$ changes is hardly observed.

Thus, since the frequency component caused by the micro vibration of the heartbeat is extracted, the improvement of robustness and the reduction of the effects of disturbances can be ensured. Then, since the respiration rate is calculated based on the frequency component of the respiration phase change with the heartbeat phase change removed, the avoidance of multiple times counting and the extension of a detection range can be ensured in consideration of a reflected signal from a chest without considering a reflected signal from an abdomen. Furthermore, since the data that serves as a basis for calculating the respiration rate is different from the data that serves as a basis for calculating the heart rate (described above), the separation of respiration and heartbeat can be ensured.

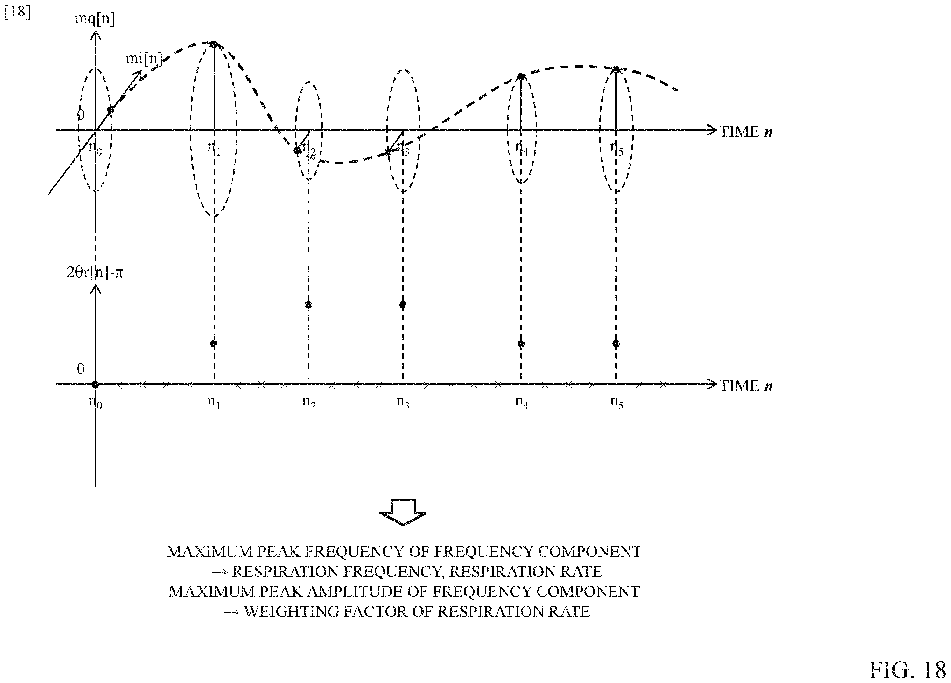

Fig. 18 illustrates a specific example of the respiration detection processing of this disclosure. The respiration phase extraction unit 5 extracts amplitude peaks caused by the micro vibration of the heartbeat from complex multiplication of the positive and negative frequency components $|A|^2 e^{j(2\theta r-\pi)}$ and extracts the respiration phase change $2\theta_r-\pi$ at the amplitude peaks while applying zero padding between the amplitude peaks without extracting the respiration phase change $2\theta_r-\pi$ (Step S5). The respiration rate calculation unit 6 calculates an average respiration rate with increasing a weight of the respiration rate as a maximum peak amplitude of the frequency component of the respiration phase change $2\theta_r-\pi$ increases (Step S6).

In Fig. 18, at the peak time $n_0$, an amplitude peak is present, and the respiration phase change $2\theta_r-\pi$ = 0 is extracted. At the peak time $n_1$ an amplitude peak is present, and the respiration phase change $2\theta_r-\pi = \pi/2$ is extracted. At the peak time $n_2$, an amplitude peak is present, and the respiration phase change $2\theta_r-\pi = \pi$ is extracted. At the peak time $n_3$, an amplitude peak is present, and the respiration phase change $2\theta_r-\pi = \pi$ is extracted. At the peak time $n_4$, an amplitude peak is present, and the respiration phase change $2\theta_r-\pi = \pi/2$ is extracted. At the peak time $n_5$, an amplitude peak is present, and the respiration phase change $2\theta_r-\pi = \pi/2$ is extracted. Between the peak times $n_0$, $n_1$, $n_2$, $n_3$, $n_4$, and $n_5$, amplitude peaks are not present, and I = Q = 0 is applied as zero padding.

In Fig. 18, based on a maximum peak frequency of the frequency component of the respiration phase change $2\theta_r-\pi$, the respiration frequency and therefore the respiration rate are calculated. Then, based on the maximum peak amplitude of the frequency component of the respiration phase change $2\theta_r-\pi$, the weighting factor of the respiration rate is calculated. Here, similarly to a case when the heart rate is calculated, even when the respiration rate is calculated, the weight of the respiration rate according to the maximum peak amplitude may be calculated, and the forgetting coefficient $\lambda$ may be set according to the weighting factor.

Thus, in consideration of the fluctuation of the heartbeat, information on the respiration phase change is used at heartbeat amplitude peak time while the information on the respiration phase change is not used between the heartbeat amplitude peaks, and therefore, the calculation of the respiration rate can be performed with high accuracy. Here, instead of performing a frequency conversion of the respiration phase change $\theta_r$, a frequency conversion of the respiration phase change $2\theta_r-\pi$ is performed ($2\theta_r$ is doubled compared with $\theta_r$), and therefore, the maximum peak frequency of the frequency component can be calculated with high accuracy. Then, since the respiration rate is weighted according to the magnitude of the maximum peak amplitude of the frequency component of the respiration phase change, the calculation of the respiration rate can be performed with high accuracy.

In this embodiment, the respiration phase change $2\theta_r-\pi$ caused by the micro vibration of the respiration is extracted from the frequency component (mi[n], mq[n]). Simultaneously with this, a respiration amplitude change $|A|^2$ caused by the micro vibration of the respiration is also extracted from the frequency component (mi[n], mq[n]). As a reason for this, complex multiplication of the positive and negative frequency components $|A|^2 e^{j(2\theta r-\pi)}$ are calculated.

As a modification, one of the respiration phase change and the respiration amplitude change caused by the micro vibration of the respiration may be extracted from the frequency component (mi[n], mq[n]). As another example, one or both of the respiration phase change and the respiration amplitude change caused by the micro vibration of the respiration may be extracted from the frequency component (ri[n], rq[n]), (li[n], lq[n]), or b[n].

(Result of Heartbeat and Respiration Detection Processing of This Disclosure)

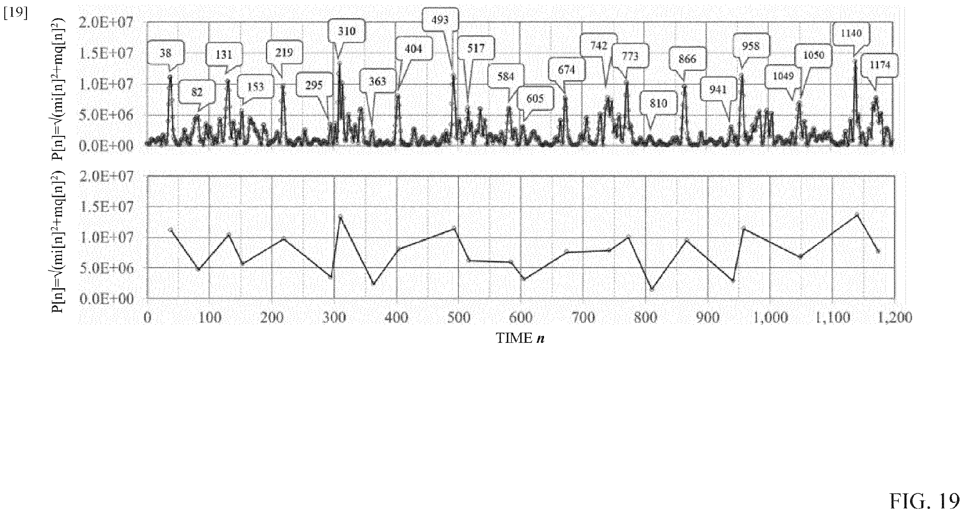

Fig. 19 and Fig. 20 illustrate results of the heartbeat detection processing of this disclosure. The upper stage of Fig. 19 illustrates a temporal change of an amplitude $P[n] = \sqrt{(mi[n]^2 + mq[n]^2)}$ caused by the micro vibration of the heartbeat. The lower stage of Fig. 19 illustrates peak times of the amplitude $P[n] = \sqrt{(mi[n]^2 + mq[n]^2)}$ caused by the micro vibration of the heartbeat. Here, the processing illustrated in Fig. 8 and Fig 9 is performed.

The left section of Fig. 20 illustrates the weight of the heartbeat interval $\tau_a$. The middle section of Fig. 20 illustrates the weight of the heartbeat interval $\tau_b$. The right section of Fig. 20 illustrates the weight of the heartbeat interval $\tau_c$. Here, in the heartbeat interval of $0 < \tau_a < 50$ and the heartbeat interval of $150 < \tau_c < 200$, the upper limit and the lower limit of the heartbeat interval are exceeded and the heartbeat interval is ignored. Then, at the heartbeat interval $\tau_a \approx 90$ and the heartbeat interval $\tau_b \approx 90$, clusters of the weight of the heartbeat interval are extracted. Accordingly, 61 bpm can be calculated as the heart rate. Note that when the weight is low, the detection result may be ignored, or the detection result may be warned. In addition, when the cluster is extracted, K-means clustering, DBSCAN, or the like may be applied.

In the lower section of Fig. 20, different from the left section, the middle section, and the right section of Fig. 20, the weights of the heartbeat intervals $\tau_a$, $\tau_b$, and $\tau_c$ are not illustrated separately, but the weights of the

heartbeat intervals $\tau_a$, $\tau_b$, and $\tau_c$ are synthesized and illustrated. Here, clusters are extracted from this two-dimensional data, and outliers are removed. For the clusters, a weighted average of the heartbeat interval is calculated, making the accuracy of the heart rate higher.

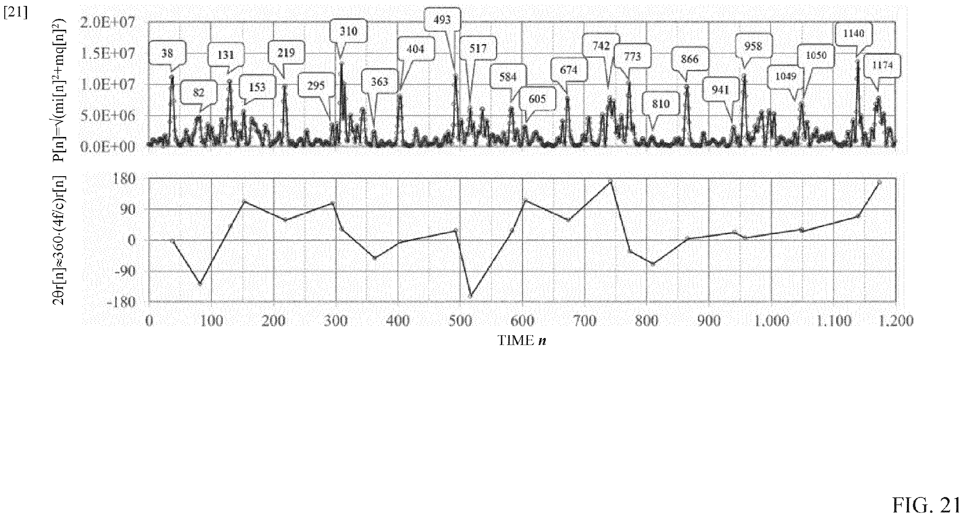

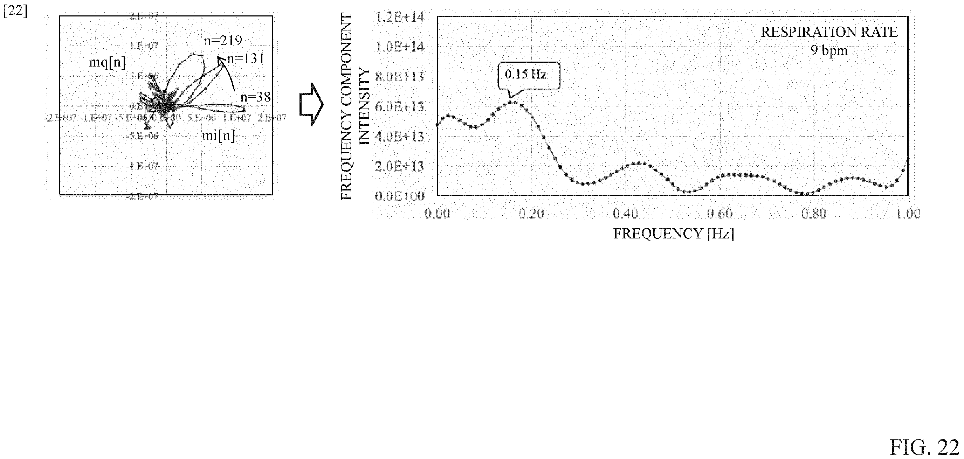

Fig. 21 and Fig. 22 illustrate results of the respiration detection processing of this disclosure. The upper stage of Fig. 21 illustrates the temporal change of the amplitude $P[n] = \sqrt{(mi[n]^2 + mq[n]^2)}$ caused by the micro vibration of the heartbeat. The lower stage of Fig. 21 illustrates a respiration phase change $2\theta_r[n]-\pi = 360 \cdot (4f/c)r[n]$ ($r[n]$ is a distance) of the amplitude peaks. Here, the processing illustrated in Fig. 15 and Fig 18 is performed.

The left section of Fig. 22 illustrates a constellation of the amplitude peaks. The right section of Fig. 22 illustrates a frequency conversion of the respiration phase change $2\theta_r[n]-\pi$. Here, a monotonous change of the respiration phase change $2\theta_r[n]-\pi$ is detected from n = 38 through n = 131 to n = 219. Then, the maximum peak frequency of the frequency conversion is calculated at the frequency component = 0.15 Hz of the frequency conversion. Accordingly, 9 bpm can be calculated as the respiration rate.

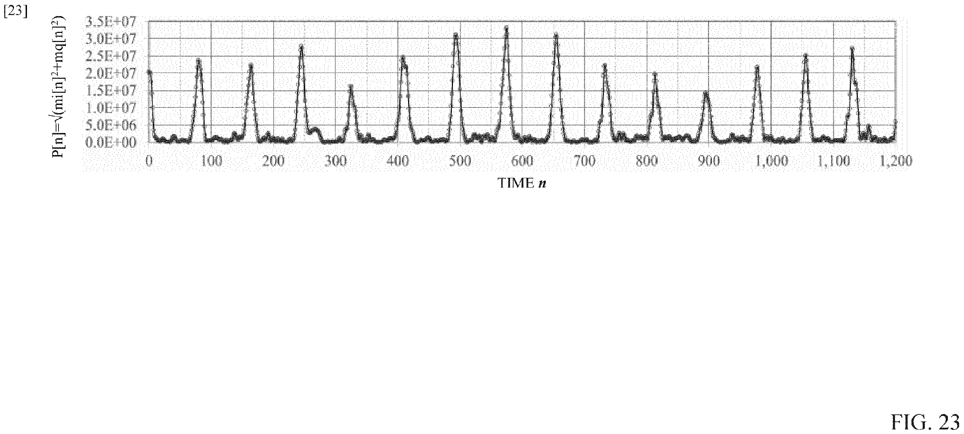

Fig. 23 also illustrates a result of the respiration detection processing of this disclosure. Fig. 23 illustrates the temporal change of the amplitude $P[n] = \sqrt{(mi[n]^2 + mq[n]^2)}$ caused by the micro vibration of the heartbeat. Here, the amplitude $P[n]$ caused by the micro vibration of the heartbeat forms peaks very regularly. In this case, even when only the respiration amplitude change $|A|^2$ caused by the micro vibration of the respiration is extracted, the respiration rate can be calculated with high accuracy to some extent. However, the amplitude $P[n]$ caused by the micro vibration of the heartbeat does not necessarily form peaks regularly. Even in this case, by extracting the respiration phase change $2\theta_r-\pi$ caused by the micro vibration of the respiration, the respiration rate can be calculated with high accuracy regardless of a situation.

INDUSTRIAL APPLICABILITY

The heartbeat and respiration detection device and the heartbeat and respiration detection program of this disclosure can calculate a heart rate and a respiration rate while enabling the alleviation of burdens on nurses and infection risk reduction based on a radar signal (an ultrasound signal may be included) reflecting off a body surface.

REFERENCE SIGNS LIST

| S | Spectrogram |
| B | Band-pass filter result |
| P | Patient |
| R | Radar transmission/reception device, ultrasound transmission/reception device |
| M | Heartbeat and respiration detection device |
| Wa, Wb, Wc, Wd | Heartbeat observation time window |
| WO, W1, W2, W3 | Peak detection time window |
| W | Peak detection time window |
| 1 | Heartbeat component extraction unit |
| 2 | Heartbeat component multiplication unit |
| 3 | Heartbeat peak extraction unit |
| 4 | Heart rate calculation unit |
| 5 | Respiration phase extraction unit |
| 6 | Respiration rate calculation unit |

**Claims**

1. A heartbeat and respiration detection device comprising:

   a heartbeat component extraction unit that extracts a frequency component caused by a micro vibration of heartbeat from a radar signal or an ultrasound signal reflecting off a body surface over a predefined heartbeat observation time window;
   a heartbeat peak extraction unit that extracts an amplitude peak from the frequency component caused by the micro vibration of the heartbeat and extracts one characteristic micro vibration out of a plurality of characteristic micro vibrations in one heartbeat; and
   a heart rate calculation unit that calculates a heart rate based on a time interval of the amplitude peaks or a count of the amplitude peaks extracted within a predetermined time.

2. The heartbeat and respiration detection device according to claim 1, wherein
   the heartbeat component extraction unit extracts the frequency component caused by the micro vibration of the heartbeat over the predefined heartbeat observation time window including a plurality of characteristic micro vibrations in one heartbeat.

3. The heartbeat and respiration detection device according to claim 1 or 2, wherein
   the heartbeat peak extraction unit extracts a maximum amplitude peak of the amplitude peaks from the frequency component caused by the micro vibration of the heartbeat in a predefined peak detection time window.

4. The heartbeat and respiration detection device according to claim 3, wherein
the heartbeat peak extraction unit moves the predefined peak detection time window so as to extract the maximum amplitude peak in a time domain excluding vicinities of both ends of the predefined peak detection time window.

5. The heartbeat and respiration detection device according to claim 3 or 4, wherein
the heart rate calculation unit calculates a heart rate based on a time interval of the maximum amplitude peaks extracted in an adjacent or non-adjacent predefined peak detection time window.

6. The heartbeat and respiration detection device according to any one of claims 1 to 5, wherein
the heart rate calculation unit calculates an average heart rate with increasing a weight of a time interval of the amplitude peaks as an amplitude value of the amplitude peak increases.

7. The heartbeat and respiration detection device according to any one of claims 1 to 5, wherein
the heart rate calculation unit calculates a heart rate based on clustering of two-dimensional data constituted of a time interval of the amplitude peaks and a weight of the time interval with increasing the weight of the time interval of the amplitude peaks as an amplitude value of the amplitude peak increases.

8. A heartbeat and respiration detection device comprising:

    a heartbeat component multiplication unit that extracts positive and negative frequency components caused by a micro vibration of heartbeat from a radar signal or an ultrasound signal reflecting off a body surface and performs complex multiplication of the positive and negative frequency components;
    a respiration phase extraction unit that extracts a respiration phase change caused by a micro vibration of respiration, with a heartbeat phase change caused by a micro vibration of heartbeat removed, from complex multiplication of the positive and negative frequency components; and
    a respiration rate calculation unit that calculates a respiration rate based on a frequency component of the respiration phase change.

9. The heartbeat and respiration detection device according to claim 8, wherein
the respiration phase extraction unit extracts amplitude peaks caused by a micro vibration of heartbeat from complex multiplication of the positive and negative frequency components and extracts the respiration phase change at the amplitude peak while applying zero padding between the amplitude peaks without extracting the respiration phase change.

10. The heartbeat and respiration detection device according to claim 8 or 9, wherein
the respiration rate calculation unit calculates an average respiration rate with increasing a weight of a respiration rate as a maximum peak amplitude of the frequency component of the respiration phase change increases.

11. A heartbeat and respiration detection program for causing a computer to execute each processing step corresponding to each processing unit of the heartbeat and respiration detection device according to any one of claims 1 to 10.

FIG. 1

AMPLITUDE

HEARTBEAT
(1)　　　　　　　　(2)　　　　　　　　　　(3)　　　　　　　(4)
FIRST SOUND　　SECOND SOUND　　FIRST SOUND　　SECOND SOUND

TIME

FIG. 2

FIG. 3

[4]

HEARTBEAT AND RESPIRATION DETECTION DEVICE M

R

P

RADAR TRANSMISSION/ RECEPTION DEVICE

OR

ULTRASOUND TRANSMISSION/ RECEPTION DEVICE

1 HEARTBEAT COMPONENT EXTRACTION UNIT

2 HEARTBEAT COMPONENT MULTIPLICATION UNIT

3 HEARTBEAT PEAK EXTRACTION UNIT

4 HEART RATE CALCULATION UNIT

5 RESPIRATION PHASE EXTRACTION UNIT

6 RESPIRATION RATE CALCULATION UNIT

FIG. 4

FIG. 5

EP 4 413 922 A1

[6]

```
            ┌────────────────────────────────────────┐
            │      START HEART RATE CALCULATION      │
            └────────────────────────────────────────┘
                              │
                              ▼                    S1
    ┌──────────────────────────────────────────────────┐
    │      EXTRACT (li[n], lq[n]), (ri[n], rq[n]), OR b[n]  │
    │            OVER PREDEFINED HEARTBEAT             │
    │            OBSERVATION TIME WINDOW              │
    └──────────────────────────────────────────────────┘
                              │
                              ▼                    S2
    ┌──────────────────────────────────────────────────┐
    │              EXTRACT AMPLITUDE PEAKS             │
    │     FROM (li[n], lq[n]), (ri[n], rq[n]), b[n],      │
    │        OR (mi[n], mq[n]) OBTAINED BY           │
    │            COMPLEX MULTIPLICATION             │
    │         EXTRACT ONE CHARACTERISTIC            │
    │                 MICRO VIBRATION                  │
    │   OUT OF A PLURALITY OF CHARACTERISTIC       │
    │   MICRO VIBRATIONS IN ONE HEARTBEAT        │
    └──────────────────────────────────────────────────┘
                              │
                              ▼                    S3
    ┌──────────────────────────────────────────────────┐
    │              CALCULATE HEART RATE              │
    │  BASED ON TIME INTERVAL OF AMPLITUDE PEAKS  │
    │  OR EXTRACTION COUNT OF AMPLITUDE PEAKS     │
    │         WITHIN PREDETERMINED TIME            │
    └──────────────────────────────────────────────────┘
                              │
                              ▼
            ┌────────────────────────────────────────┐
            │      END HEART RATE CALCULATION        │
            └────────────────────────────────────────┘
```

FIG. 6

[7]

FIG. 7

EP 4 413 922 A1

FIG. 8

EP 4 413 922 A1

[9]

60/t bpm

WEIGHT ACCORDING TO AMPLITUDE VALUE

$\tau_a \rightarrow$ SELECTED    $\tau_b \rightarrow$ IGNORED    $\tau_c \rightarrow$ IGNORED

60/2t bpm

WEIGHT ACCORDING TO AMPLITUDE VALUE

$\tau_b \rightarrow$ SELECTED

60/3t bpm

WEIGHT ACCORDING TO AMPLITUDE VALUE

$\tau_c \rightarrow$ SELECTED

0        t        2t        3t        4t

HEARTBEAT INTERVAL [sec]

FIG. 9

EP 4 413 922 A1

[10]

FIG. 10

[11]

FIG. 11

EP 4 413 922 A1

[12]

FIG. 12

AMPLITUDE

W0

CANDIDATE FIRST SOUND

THRESHOLD

$n_0$

t/2 — t/2

---

AMPLITUDE

W0

NON-CANDIDATE FIRST SOUND

CANDIDATE SECOND SOUND

THRESHOLD

$n_1$

t/2 — t/2

---

AMPLITUDE

W0

NON-CANDIDATE FIRST SOUND

CANDIDATE SECOND SOUND

NON-CANDIDATE FIRST SOUND

THRESHOLD

$n_2$

t/2 — t/2

---

AMPLITUDE

W0

NON-CANDIDATE FIRST SOUND

MAXIMUM SECOND SOUND

NON-CANDIDATE FIRST SOUND

CANDIDATE SECOND SOUND

THRESHOLD

$n_3$

t/2 — t/2

EXTRACT MAXIMUM AMPLITUDE PEAK IN WINDOW W1

W0    W1    W2    W3

MAXIMUM $p_0$    MAXIMUM $p_1$    MAXIMUM $p_2$    MAXIMUM $p_3$

AMPLITUDE

$t/2$   $n_0$   $t/2$    $t/2$   $n_1$   $t/2$   TIME   $t/2$   $n_2$   $t/2$    $t/2$   $n_3$   $t/2$

HEARTBEAT INTERVAL $\tau_a = n_1 - n_0$

WEIGHTING FACTOR $\sqrt{(p_0 p_1)}$

HEARTBEAT INTERVAL $\tau_b = n_2 - n_0$

WEIGHTING FACTOR $\sqrt{(p_0 p_2)}$

HEARTBEAT INTERVAL $\tau_c = n_3 - n_0$

WEIGHTING FACTOR $\sqrt{(p_0 p_3)}$

60/t bpm

$\tau_a \rightarrow$ SELECTED    $\tau_b \rightarrow$ IGNORED    $\tau_c \rightarrow$ IGNORED

WEIGHT ACCORDING TO AMPLITUDE VALUE

0    t    2t    3t    4t

HEARTBEAT INTERVAL [sec]

FIG. 13

EP 4 413 922 A1

EP 4 413 922 A1

[14]

$$\text{START RESPIRATION RATE CALCULATION}$$

S4

EXTRACT (li[n], lq[n]) AND (ri[n], rq[n])
CALCULATE (mi[n], mq[n])
OBTAINED BY COMPLEX MULTIPLICATION

S5

EXTRACT PHASE CHANGE
CAUSED BY MICRO VIBRATION OF RESPIRATION,
WITH PHASE CHANGE
CAUSED BY MICRO VIBRATION OF HEARTBEAT REMOVED,
FROM (mi[n], mq[n]) OBTAINED BY
COMPLEX MULTIPLICATION

S6

CALCULATE RESPIRATION RATE
BASED ON FREQUENCY COMPONENT
OF RESPIRATION PHASE CHANGE

END RESPIRATION RATE CALCULATION

FIG. 14

FIG. 15

EP 4 413 922 A1

FIG. 16

[17]

AFTER COMPLEX MULTIPLICATION

RESPIRATION PHASE CHANGE

WITHOUT HEARTBEAT PHASE CHANGE

FIG. 17

MAXIMUM PEAK FREQUENCY OF FREQUENCY COMPONENT
→ RESPIRATION FREQUENCY, RESPIRATION RATE
MAXIMUM PEAK AMPLITUDE OF FREQUENCY COMPONENT
→ WEIGHTING FACTOR OF RESPIRATION RATE

FIG. 18

FIG. 19

EP 4 413 922 A1

[20]

FIG. 20

EP 4 413 922 A1

FIG. 21

EP 4 413 922 A1

FIG. 22

$$P[n]=\sqrt{(mi[n]^2+mq[n]^2)}$$

TIME $n$

EP 4 413 922 A1

FIG. 23

## INTERNATIONAL SEARCH REPORT

International application No.

**PCT/JP2022/036795**

### A. CLASSIFICATION OF SUBJECT MATTER

*A61B 5/11*(2006.01)i; *A61B 5/0245*(2006.01)i; *A61B 5/08*(2006.01)i; *A61B 5/113*(2006.01)i
FI:　A61B5/11 110; A61B5/0245 100A; A61B5/08; A61B5/113

According to International Patent Classification (IPC) or to both national classification and IPC

### B. FIELDS SEARCHED

Minimum documentation searched (classification system followed by classification symbols)

A61B5/00-5/398; A61B8/02; G01S13/00-13/95

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2017-006540 A (FUJITSU LIMITED) 12 January 2017 (2017-01-12) claim 1, paragraphs [0038]-[0075], fig. 9-21 | 1-5, 11 |
| Y | | 6–7 |
| A | | 8-10 |
| Y | JP 2018-102905 A (MURATA MANUFACTURING CO., LTD.) 05 July 2018 (2018-07-05) paragraphs [0089]-[0092] | 6–7 |
| A | JP 2012-239748 A (KABUSHIKI KAISHA TOYOTA CHUO KENKYUSHO) 10 December 2012 (2012-12-10) abstract, paragraphs [0045]-[0055], fig. 1, 3-4 | 1-11 |
| A | JP 2011-015887 A (MITSUBISHI ELECTRIC CORPORATION) 27 January 2011 (2011-01-27) entire text, all drawings | 1–11 |
| A | US 2019/0008459 A1 (STICHTING IMEC NEDERLAND) 10 January 2019 (2019-01-10) entire text, all drawings | 1–11 |

☑ Further documents are listed in the continuation of Box C.  ☑ See patent family annex.

| | |
|---|---|
| *　　Special categories of cited documents:<br>"A"　document defining the general state of the art which is not considered to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search | Date of mailing of the international search report |
|---|---|
| **28 November 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/036795**

| C. | DOCUMENTS CONSIDERED TO BE RELEVANT | |
|---|---|---|
| Category* | Citation of document, with indication, where appropriate, of the relevant passages | Relevant to claim No. |
| A | WO 2019/044195 A1 (MURATA MANUFACTURING CO., LTD.) 07 March 2019 (2019-03-07)<br>    entire text, all drawings | 8-11 |
| A | CN 106175731 A (SHANGHAI JIAOTONG UNIVERSITY) 07 December 2016 (2016-12-07)<br>    entire text, all drawings | 8-11 |

Form PCT/ISA/210 (second sheet) (January 2015)

# EP 4 413 922 A1

| INTERNATIONAL SEARCH REPORT | International application No. |
|---|---|
| | **PCT/JP2022/036795** |

**Box No. III      Observations where unity of invention is lacking (Continuation of item 3 of first sheet)**

This International Searching Authority found multiple inventions in this international application, as follows:

Document 1: JP 2017-006540 A (FUJITSU LTD.) 01 December 2017 (2017-01-12)
        claim 1, paragraphs [0038]-[0075], fig. 9-21
        (Family: none)

Document 3: JP 2012-239748 A (TOYOTA CENTRAL R&D LABS., LTD.) 10 December 2012 (2012-12-10)
        abstract, paragraphs [0045]-[0055], fig. 1, 3-4
        (Family: none)

Invention 1: claims 1-7, 11
    Claims 1-7 and 11 are classified as invention 1 due to having the special technical feature of "a heartbeat respiration detection device characterized by comprising: a heartbeat component extraction unit that extracts, from an ultrasonic signal or radar signal reflected at a body surface, a frequency component from micro-vibration of a heartbeat across a prescribed heartbeat measurement time; a heartbeat peak extraction unit that extracts an amplitude peak from the frequency component from the micro-vibration of the heartbeat, and that extracts one characteristic micro-vibration among a plurality of characteristic micro-vibrations in one round of heartbeat; and a heart rate calculation unit that calculates a heart rate on the basis of the number of extractions of the amplitude peak in a time interval or the amplitude peak or within a predetermined time."

(Invention 2) Claims 8-10
    Claims 8-10 share, with claim 1 classified as invention 1, the technical feature of "extracts, from an ultrasonic signal or radar signal reflected at a body surface, a frequency component from micro-vibration of a heartbeat." However, this technical feature does not make a contribution over the prior art in light of the content disclosed in documents 1 and 3, and thus this technical feature cannot be said to be a special technical feature. Furthermore, there are no other identical or corresponding special technical features between these inventions.
    Additionally, claims 8-10 are not dependent claims of claim 1. Moreover, claims 8-10 are not substantially identical to or similarly closely related to any of the claims classified as invention 1.
    Therefore, claims 8-10 cannot be classified as invention 1.
    Claims 8-10 are classified as invention 2 due to having the special technical feature of "a heartbeat respiration detection device characterized by comprising: a heartbeat component multiplication unit that extracts, form an ultrasonic signal or radar signal reflected at a body surface, positive and negative frequency components from a micro-vibration of a heartbeat, and performs complex multiplication of the positive and negative frequency components; a respiratory phase extraction unit that extracts, from the positive and negative frequency components subjected to complex multiplication, a respiratory phase change from a micro-vibration of respiration, from which a heartbeat phase change from micro-vibration of the heartbeat has been removed; and a respiration rate calculation unit that calculates a respiration rate on the basis of the frequency component of the respiratory phase change."

Form PCT/ISA/210 (continuation of first sheet) (January 2015)

**39**

## INTERNATIONAL SEARCH REPORT

International application No.

**PCT/JP2022/036795**

| Box No. III | Observations where unity of invention is lacking (Continuation of item 3 of first sheet) |
|---|---|

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

INTERNATIONAL SEARCH REPORT
Information on patent family members

International application No.

**PCT/JP2022/036795**

| Patent document cited in search report | | | Publication date (day/month/year) | Patent family member(s) | | | Publication date (day/month/year) |
|---|---|---|---|---|---|---|---|
| JP | 2017-006540 | A | 12 January 2017 | (Family: none) | | | |
| JP | 2018-102905 | A | 05 July 2018 | US | 2018/0092564 | A1 | |
| | | | | paragraphs [0091]-[0092] | | | |
| | | | | EP | 3305180 | A1 | |
| | | | | CN | 107913060 | A | |
| JP | 2012-239748 | A | 10 December 2012 | (Family: none) | | | |
| JP | 2011-015887 | A | 27 January 2011 | FR | 2947712 | A1 | |
| US | 2019/0008459 | A1 | 10 January 2019 | EP | 3424418 | A1 | |
| WO | 2019/044195 | A1 | 07 March 2019 | US | 2020/0260974 | A1 | |
| | | | | entire text, all drawings | | | |
| | | | | CN | 111050639 | A | |
| CN | 106175731 | A | 07 December 2016 | (Family: none) | | | |

Form PCT/ISA/210 (patent family annex) (January 2015)

**EP 4 413 922 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019129996 A